Europäisches Patentamt

(19) European Patent Office

Office européen des brevets

(11) EP 0 625 901 B1

# (12) FASCICULE DE BREVET EUROPEEN

(45) Date de publication et mention
de la délivrance du brevet:
**04.11.1998 Bulletin 1998/45**

(21) Numéro de dépôt: **94902838.5**

(22) Date de dépôt: **20.12.1993**

(51) Int. Cl.$^6$: **A61K 9/70**

(86) Numéro de dépôt international:
**PCT/FR93/01272**

(87) Numéro de publication internationale:
**WO 94/14425 (07.07.1994 Gazette 1994/15)**

(54) **FILMS A BASE DE COPOLYMERES, LEURS APPLICATIONS DANS DES SYSTEMES TRANSDERMIQUES ET LEURS PROCEDES DE PREPARATION**

FILME AUF COPOLYMERENBASIS, DEREN VERWENDUNGEN IN TRANSDERMALEN SYSTEMEN UND DEREN HERSTELLUNGSVERFAHREN

COPOLYMER-BASED FILMS, APPLICATIONS THEREOF IN TRANSDERMAL SYSTEMS AND PROCESSES FOR THEIR PREPARATION

(84) Etats contractants désignés:
**AT BE CH DE DK ES FR GB GR IE IT LI LU MC NL PT SE**

(30) Priorité: **21.12.1992 FR 9215377**

(43) Date de publication de la demande:
**30.11.1994 Bulletin 1994/48**

(73) Titulaire: **BIO HOLDING**
**91741 Massy Cédex (FR)**

(72) Inventeurs:
• **ISTIN, Michel**
**F-91190 Gif-sur-Yvette (FR)**

• **GROGNET, Jean-Marc**
**F-91120 Palaiseau (FR)**
• **DARNEZ, Charles**
**F-33200 Bordeau-Cauderan (FR)**

(74) Mandataire: **Ores, Irène et al**
**CABINET ORES**
**6, Avenue de Messine**
**75008 Paris (FR)**

(56) Documents cités:
**EP-A- 0 078 184          EP-A- 0 379 045**
**WO-A-92/20377          FR-A- 2 250 793**

## Description

La présente invention est relative à des films à base de copolymères, aptes à être utilisés en tant que matrices de principes actifs dans des systèmes transdermiques, aux systèmes transdermiques contenant lesdits films ainsi qu'au procédé de préparation desdits films et copolymères.

L'administration transdermique peut être, dans certains cas, une voie privilégiée d'administration de médicaments ; en effet, une telle voie permet d'optimiser l'effet systémique ou l'effet topique, d'augmenter l'efficacité thérapeutique, de réduire les effets indésirables et d'éviter, dans le cas d'une action systémique, l'effet de premier passage hépatique.

De manière générale, les systèmes transdermiques sont des formes destinées à permettre le passage de principes actifs, incorporés dans un réservoir, a travers la peau, soit pour avoir une action systémique, soit pour obtenir une action plus locale, par fixation dans les tissus sous-jacents de la peau.

De tels systèmes peuvent prendre essentiellement deux formes :

. une poche servant de réservoir, limitée sur l'une de ses surfaces, par une membrane hemi-perméable adhésive, placée contre la peau et qui contrôle la libération et le passage du principe actif vers la peau ; dans ce cas, la membrane contrôle la libération des principes actifs et donc, dans le cas d'une action systémique, leur concentration sanguine ;

. une matrice polymérique, servant de réservoir, et associée à un adhésif, qui contrôle la libération vers la peau du principe actif ; cette forme comporte deux variantes : (1) soit la matrice polymérique est constituée d'un adhésif, chargé de fixer le système sur la peau, qui joue le rôle de réservoir, (2) soit la matrice polymérique et l'adhésif sont séparés et seule la matrice polymérique joue le rôle de réservoir et contrôle la libération du principe actif, l'adhésif n'ayant pour fonction, que de faire tenir le système sur la peau, sans entraver le passage du principe actif.

La vitesse de libération est donc contrôlée soit par la membrane placée entre le réservoir et la peau, soit par le réservoir lui-même (alors généralement dénommé matrice).

Compte-tenu de ce qui précède, de nombreux facteurs doivent être considérés pour mettre au point un système transdermique.

Il est, en particulier, important de disposer d'un réservoir qui permette une libération régulière du principe actif, assurant une concentration sanguine constante ou une concentration suffisante dans les tissus sous-jacents, pendant une durée suffisamment importante (plusieurs jours), de manière à éviter des applications trop fréquentes de tels systèmes.

De nombreux systèmes ont été proposés dans l'Art antérieur et préconisent notamment la présence d'une membrane qui contrôle la libération des principes actifs et donc, en particulier, leur concentration sanguine.

Dans le cas où la peau n'est pas suffisamment perméable-au principe actif, des stimulateurs de la perméabilité sont associés auxdits principes actifs.

La Demande EP 399 765, au nom de ADVANCED POLYMER SYSTEMS, INC. décrit notamment un système transdermique incluant un support imperméable et une matrice, en matériel adhésif approprié tel qu'un polyisobutylène ou un acrylique et qui inclut une pluralité de particules polymériques ; ces particules polymériques incluent au moins un stimulateur de pénétration (ou stimulateur de perméabilité), tandis que le principe actif à administrer peut être présent soit sous forme solide, soit sous forme liquide, dans ladite matrice ou dans lesdites particules polymériques.

Il est spécifié que le choix de la matrice et du stimulateur de perméabilité sont liés au principe actif ; en particulier, lorsque le principe actif est du lévonorgestrel, la matrice, qui doit être à la fois un réservoir de principes actifs et de particules polymériques est de l'EVA et le stimulateur est un mélange acétate d'éthyle/éthanol, et est inclus dans lesdites particules polymériques, qui peuvent être préparées à partir de monomères insaturés monoéthyléniques (esters d'acide acrylique ou méthacrylique).

La Demande Internationale WO 90/07940, au nom de NOVEN PHARMACEUTICALS, INC., décrit un système transdermique qui comprend une couche adhésive qui inclut le principe actif et un support imperméable.

La couche adhésive est multipolymérique et comprend avantageusement le principe actif, un multipolymère contenant de l'EVA et incluant un polymère acrylique (adhésif), un polymère élastomérique (gomme) et un agent tackifiant.

Les copolymères EVA (acétate de vinyle : entre 4 et 80 % en poids et éthylène : 15 à 90 %) peuvent être soit des copolymères, soit des terpolymères acide acrylique/éthylène/acétate de vinyle.

Le rapport EVA/acrylate est de préférence compris entre 20:1 et 1:20 en poids. Un agent réticulant peut éventuellement être utilisé. La composition comprend avantageusement entre 3 et 20 % d'EVA, entre 25 et 70 % d'acrylate et entre 2 et 20 % de gomme.

La Demande Internationale WO 91/16085, au nom de ALZA CORPORATION, décrit des adhésifs à base de polyisobutylène (PIB) pour système transdermique.

Elle décrit en particulier un système transdermique comprenant :

- un réservoir contenant de la nicotine et comprenant de 60 à 95 % en poids d'un copolymère EVA ayant un contenu en acétate de vinyle d'environ 40 %,
- une couche adhésive, comprenant de la nicotine dissoute dans un polymère consistant essentiellement en un mélange de PIB de haut poids moléculaire et de PIB de bas poids moléculaire,
- et un agent de contrôle de la libération du principe actif, disposé entre le réservoir et l'adhésif.

Le Brevet européen 318 385, au nom de PIERRE FABRE MEDICAMENT, décrit et revendique un dispositif multilamellaire pour l'administration transdermique de trinitrine comportant successivement un support réalisé en un matériau souple et occlusif (film thermoplastique coextrudé type EVA/PVDC/EVA), un réservoir adhésif actif à base de polymères de type acrylique ou silicone, incorporant le principe actif, une membrane de transfert du principe actif, de type polyuréthane, une interface adhésive hypoallergénique auto-alimentée par le réservoir en principe actif, un protecteur réalisé en un film rigide ou semi-rigide revêtu d'un anti-adhésif (papiers simples ou aluminisés, polyesters ou PVC), caractérisé en ce qu'il est constitué à partir d'un premier module comprenant le support, le réservoir adhésif actif et un papier de protection temporaire et d'un deuxième module comportant la membrane de transfert, l'interface adhésive et le protecteur anti-adhérent, les deux modules étant collés l'un sur l'autre après l'enlèvement du papier de protection du premier module par pressage du réservoir actif contre la membrane perméable du transfert du principe actif.

La Demande de Brevet européen 328 806, au nom de PACO PHARMACEUTICAL SERVICES, décrit un système transdermique d'estradiol qui comprend un support imperméable, une matrice comprenant de 60 à 95 % d'un polymère adhésif (multipolymère acétate de vinyle/acrylate), de 5 à 20 % d'un solvant (propylène glycol et dérivés), de 0,2 à 4 % d'un stimulateur de pénétration dans la peau (ester de polyoxyéthylène, mono-9-octadécénoate poly(oxy-1,2-éthanediyl) sorbitane) et de 0,5 à 5 % d'un principe actif (oestrogène et dérivés).

La Demande Internationale PCT WO 92/20377 décrit également un dispositif transdermique à base d'EVA, comprenant un stimulateur de la perméabilité des médicaments à travers la peau (monolinoléate de glycérol).

L'ensemble de ces documents montre la variété des dispositifs transdermiques, comportant notamment une matrice polymérique à base d'EVA ; toutefois ces dispositifs de l'Art antérieur présentent un certain nombre d'inconvénients :

- l'adhésif joue le rôle de réservoir, ce qui ne permet que des actions de courte durée, les charges en principe actif trop importantes entraînant une perte d'adhésivité (capacité limitée de chargement en principe actif) ;
- chacun des systèmes n'est adapté qu'à un principe actif particulier ;
- la vitesse de libération du principe actif est liée à la nature de l'adhésif.

Ces inconvénients rendent notamment difficile pour ces systèmes transdermiques, l'obtention de taux sanguins en principes actifs contrôlés, en dessous des flux limitants dûs à la peau, durant plusieurs jours.

Des systèmes de relargage contrôlé de médicaments ou de produits chimiques en milieu aqueux ont également été décrits :

- la Demande de Brevet français n° 2 250 793 décrit un substrat hydrophobe comprenant des inclusions polymérisées de substances hydrophiles, qui permettent de constituer des zones d'accumulation de substances chimiques qui pourront ensuite être délivrées (relarguées) lentement lorsque le substrat ainsi chargé (réservoir de principe actif), est mis en présence d'eau (ou d'alcool) ;
- la Demande de Brevet EP 0 078 184) décrit un dispositif de conditionnement d'engrais et/ou d'autres produits utilisables en agriculture. L'une des parois du dispositif de conditionnement, sous forme de barquette ou de tube, est constituée d'une membrane en polymère hydrophobe comportant des inclusions hydrophiles. De manière préférée, une telle membrane est un copolymère EVA/AA dans lequel le taux d'inclusions hydrophiles (AA) est de 25 à 50 %.

Un relargage contrôlé et différé d'engrais à partir de ce dispositif, n'a lieu que lors de la mise en contact du conditionnement avec de l'eau.

La présente invention s'est, pour sa part, donné pour but de pourvoir à des films aptes à être utilisés en tant que matrices de principes actifs dans des systèmes transdermiques, qui répondent mieux aux besoins de la pratique, notamment en ce qu'ils peuvent être adaptés, quel que soit le principe actif, en ce qu'ils permettent d'inclure, si nécessaire, des concentrations importantes de principes actifs, en ce qu'ils augmentent la stabilité au stockage des principes actifs et notamment des principes actifs instables et en ce qu'ils sont particulièrement bien adaptés à l'obtention de taux sanguins en principes actifs contrôlés, en dessous des flux limitants dûs à la peau, durant plusieurs jours (libération du principe actif indépendante de l'adhésif).

La présente invention s'est également donné pour but de pourvoir aux systèmes transdermiques contenant lesdits films ainsi qu'au procédé de préparation desdits films.

La présente invention a pour objet un film, apte à être utilisé comme matrice de principe actif dans un système transdermique, caractérisé en ce qu'il est constitué par un polymère hydrophobe de type éthylène/acétate de vinyle (EVA) contenant au moins un principe actif et comportant 10 à 50 %, de préférence 15 à 50 %, d'inclusions hydrophiles formées à partir d'au moins un monomère hydrophile choisi dans le groupe qui comprend l'acrylamide, le méthylol acrylamide, le diacétone acrylamide, l'acide maléique, l'acide acrylique, l'acide fumarique, l'acide itaconique, l'acrylate de propylène glycol, le méthacrylate d'éthylène glycol, le méthacrylamide, l'acide méthacrylique, le méthacrylate de propylène glycol, le méthacrylate d'hydroxyéthyle, le méthacrylate de diméthylaminoéthyle, la N-vinylpyrrolidone, l'acide vinylacétique, les acides vinylsulfoniques.

Les inclusions hydrophiles, au sens de la présente invention, correspondent aux monomères précités polymérisés, et éventuellement réticulés, et greffés sur ledit polymère hydrophobe (EVA). Le pourcentage d'inclusions hydrophiles dépend du monomère choisi.

Selon un mode de réalisation avantageux dudit film, l'EVA comprend jusqu'à 40 % d'acétate de vinyle, de préférence 14 à 22 %.

Selon un autre mode de réalisation avantageux dudit film, lesdites inclusions hydrophiles sont formées à partir d'acide acrylique.

Selon une disposition avantageuse de ce mode de réalisation, le pourcentage d'acide acrylique est compris entre 22 et 30 %.

Selon une modalité avantageuse de cette disposition, ledit copolymère comprend de l'EVA à 14-33 % d'acétate de vinyle, dans lequel 22-30 % d'acide acrylique sont greffés.

De manière préférée, ledit film comprend en tant que copolymère, de l'EVA à 14-22 % d'acétate de vinyle, dans lequel 26 % d'acide acrylique sont greffés ; un tel copolymère est ci-après dénommé EVA 18/AA 26.

L'une des compositions pondérales préférée dudit copolymère est :

| motif | % | parties |
|---|---|---|
| éthylène | 65,1 | 82 |
| acétate de vinyle | 14,3 | 18 |
| acide acrylique | 20,6 | 26 |

Ledit film conforme à l'invention peut également comprendre, en tant que copolymère, de l'EVA à 30-36 % d'acétate de vinyle, dans lequel 26 % d'acide acrylique sont greffés ; un tel copolymère est ci-après dénommé EVA 33/AA 26.

De manière inattendue, de tels copolymères et notamment le copolymère EVA 18/AA 26 en tant que matrice de principe actif, fournissent des films qui présentent simultanément :

- des propriétés mécaniques (malléabilité et élasticité, notamment) particulièrement bien adaptées à leur utilisation dans un système transdermique, sous la forme d'un film, lesquelles propriétés ne sont pas significativement altérées par l'introduction du/des principes actifs ou d'autres charges et
- une capacité de charge et de désorption du principe actif, également particulièrement bien adaptée pour une utilisation dans un système transdermique, pouvant être même supérieure à 30 % en poids par rapport au copolymère et permettant d'envisager des durées de traitement de l'ordre de sept jours.

En effet, les copolymères sélectionnés selon la présente invention permettent l'incorporation (ou chargement), à des concentrations importantes si nécessaire et la désorption convenable, de nombreux principes actifs ou de toute autre substance chimique, de manière à obtenir des taux sanguins contrôlés de ces produits ou des taux actifs dans les tissus sous-jacents ; de tels copolymères peuvent en outre être chargés en principes actifs, de différentes manières (trempage, dispersion, diffusion, immersion dans une atmosphère de vapeur saturante) et être transformables sous forme de film. De tels copolymères permettent également une stabilisation accrue des principes actifs qu'ils contiennent.

Selon un autre mode de réalisation avantageux dudit film, lesdites inclusions hydrophiles sont formées à partir d'un mélange d'acide acrylique ou d'acrylamide et de N-vinylpyrrolidone.

Conformément à ce mode de réalisation, on obtient un film constitué d'un terpolymère : EVA/AA/N-vinylpyrrolidone.

Selon encore un autre mode de réalisation avantageux dudit film, lesdites inclusions hydrophiles sont formées à partir d'acrylamide.

De manière préférée, ledit film comprend en tant que copolymère, de l'EVA à 14-22 % d'acétate de vinyle, dans lequel 26 % d'acrylamide (AAm) sont greffés (EVA 18/AAm 26).

EP 0 625 901 B1

Un tel copolymère présente un caractère hydrophile plus important que celui contenant des inclusions d'acide acrylique et permet l'incorporation de substances actives très hydrophiles.

Un pourcentage inférieur à 26 % (de l'ordre de 10-20 %) permet d'obtenir un copolymère à caractère hydrophile équivalent à celui du copolymère contenant de l'acide acrylique et dont les propriétés mécaniques sont particulièrement bien adaptées à leur utilisation dans un système transdermique.

Selon un autre mode de réalisation avantageux dudit film, son épaisseur est comprise entre 50 et 500 µm.

Selon un autre mode de réalisation avantageux dudit film, il comprend jusqu'à 40 % de principe actif.

On peut citer comme principe actif, et ce, de manière non limitative, des AINS tels que le kétoprofène, l'ibuprofène, des hormones de la reproduction telles que le 17β estradiol, éventuellement associé à un progestatif, des anticholinestérasiques tels que la physostigmine, des médicaments du système cardio-vasculaire, tels que la trinitrine, des produits utilisés dans le sevrage de l'intoxication tabagique tels que la nicotine, des médicaments de la douleur (morphine), des anti-émétiques ou des médicaments anti-migraineux tels que la dihydroergotamine, ainsi que la bromocriptine et des substances à usage local tel que l'acide salicylique.

Selon encore un autre mode de réalisation dudit film, ledit principe actif est associé à au moins un stimulateur de perméabilité, tel que alcool ou polyol.

La présente invention a également pour objet un système transdermique du type comprenant un support occlusif, un réservoir de principe actif, une interface adhésive et un film protecteur, caractérisé en ce que le réservoir de principe actif est constitué par un film tel que défini ci-dessus, dans lequel les inclusions hydrophiles sont formées à partir d'au moins un monomère hydrophile choisi dans le groupe qui comprend l'acrylamide, l'acrylate d'éthylène glycol, le méthylol acrylamide, le diacétone acrylamide, l'acide maléique, l'acide acrylique, l'acide fumarique, l'acide itaconique, l'acrylate de propylène glycol, le méthacrylate d'éthylène glycol, le méthacrylamide, l'acide méthacrylique, le méthacrylate de propylène glycol, le méthacrylate d'hydroxyéthyle, le méthacrylate de diméthylaminoéthyle, la N-vinylpyrrolidone, l'acide vinylacétique, les acides vinylsulfoniques.

De manière avantageuse, un tel système transdermique est particulièrement bien adapté au contrôle de la libération de toutes sortes de principes actifs et présente un pouvoir absorbant élevé desdits principes actifs.

De telles propriétés sont essentiellement liées aux caractéristiques dudit système, à savoir :

- le réservoir est constitué d'une matrice polymérique telle que définie ci-dessus ayant, de manière inattendue, la double fonction de réservoir et de contrôle de la libération du/des principes actifs ;
- la vitesse de libération des principes actifs est uniquement liée à la nature de la matrice polymérique constituant le réservoir : ceci entraîne que l'adhésif, associé à ladite matrice polymérique, ne modifie pas la vitesse de transfert du principe actif du réservoir vers la peau ;
- la grande adaptabilité de la matrice polymérique conforme à l'invention aux principes actifs (un ou plusieurs) ;
- l'aptitude de la matrice polymérique à être mise en forme sous forme d'un film d'épaisseur appropriée ;
- l'aptitude de la matrice polymérique à stabiliser certains des principes actifs incorporés ;
- la capacité élevée de chargement de la matrice polymérique en principe actif.

Selon un mode de réalisation préféré dudit système transdermique, il comprend :

. un film protecteur imperméable comprenant des matériaux utilisés notamment dans l'industrie alimentaire ou pharmaceutique, choisis parmi les dichlorures de polyvinylidène (PVDC), les polyéthylènes (PE), l'éthylène alcool polyvinylique, les polypropylènes, les polyesters, le polychlorotrifluoroéthylène,
. une matrice de principes actifs, constituée par un film conforme à l'invention,
. un adhésif inerte chimiquement vis-à-vis de la matrice de principes actifs et n'interférant pas dans sa capacité à désorber les principes actifs, lequel adhésif est sélectionné parmi les polymères acryliques, le polyuréthane, les silicones, l'EVA et
. une feuille de protection compatible avec l'adhésif sélectionné (polyester siliconé, par exemple).

Le caractère imperméable du film protecteur, peut être amélioré par complexage avec une feuille métallique, en aluminium par exemple, ou au moyen de tout autre procédé de métallisation.

Selon une disposition avantageuse de ce mode de réalisation, le film conforme à l'invention, matrice de principes actifs, est à base d'un copolymère EVA/AA, de préférence un copolymère EVA 18/AA 26.

Selon une autre disposition avantageuse de ce mode de réalisation, ledit film comprend en outre un stimulateur de perméabilité dudit principe actif à travers la peau, sélectionné parmi les substances non toxiques, qui ne sont pas un solvant de la matrice polymérique, telles que des alcools et des polyols (propylène glycol, PEG, ou éthanol, par exemple).

Lorsque ledit système transdermique contient au moins deux principes actifs, ceux-ci sont, soit répartis dans la masse de la matrice polymérique, soit dans un segment différent de la matrice polymérique.

La présente invention a également pour objet un procédé de préparation d'un film apte à servir de matrice de principe actif, conforme à l'invention.

Ledit procédé comprend :

(a) la mise en contact de l'EVA, sous forme de poudre, de granulé ou de film, et des monomères hydrophiles,

(b) la polymérisation réticulée desdits monomères hydrophiles et le greffage, sur l'EVA, du polymère hydrophile obtenu (copolymérisation EVA/polymère hydrophile) par irradiation, jusqu'à obtention d'un copolymère EVA/polymère hydrophile, dans lequel le taux de greffage du polymère hydrophile est compris entre 10 et 50 %,

(c) le chargement de la matrice copolymérique en principe actif et s'il y a lieu, c'est-à-dire lorsque l'EVA est sous forme de poudre ou de granulé,

(d) la mise en forme du copolymère obtenu sous la forme d'un film.

Selon un mode de mise en oeuvre avantageux dudit procédé, l'étape (b) de copolymérisation est réalisée en milieu liquide, en présence d'un inhibiteur d'homopolymérisation des monomères hydrophiles.

De manière préférée, ledit inhibiteur d'homopolymérisation est un sel ferreux tel que le sel de Mohr, lorsque, par exemple, l'acide acrylique ou l'acrylamide est utilisé comme monomère hydrophile.

Selon un autre mode de mise en oeuvre avantageux dudit procédé, l'étape (b) de copolymérisation est réalisée en milieu liquide, en présence d'agents de réticulation choisis parmi les composés polyinsaturés suivants : le méthylène bis acrylamide, le divinylbenzène, le cyanurate de triallyle, les acrylates ou les méthacrylates d'éthylène, butylène et tetraéthylène glycol, l'orthophosphate de triallyle.

De manière préférée, cette étape (b) est réalisée en irradiant pendant plusieurs heures la solution, par un rayonnement ionisant, la dose totale d'irradiation étant comprise entre 0,5 et 50 KGy, pour l'obtention du taux de greffage des polymères hydrophiles entre 10 et 50 %.

La durée d'irradiation dépend du débit de dose et de la source de rayonnement utilisée ; elle est de préférence comprise entre 2 heures et 30 heures.

Les sources du rayonnement ionisant sont notamment des générateurs de rayons X, des accélérateurs de particules et en particulier d'électrons, des sources contenant des isotopes radioactifs $^{60}$Co et $^{137}$Cs, émetteurs de rayons $\gamma$.

Selon un autre mode de mise en oeuvre avantageux dudit procédé, l'étape (c) de chargement en principe actif est réalisée avant l'étape (d) de mise en forme.

Selon un autre mode de mise en oeuvre avantageux dudit procédé, l'étape (c) de chargement en principe actif est réalisée après l'étape (d) de mise en forme.

De manière préférée, ladite étape (c) de chargement en principe actif peut être réalisée :

. soit par trempage,
. soit par dispersion dans le copolymère,
. soit par diffusion à partir d'un support imprégné en principe actif,
. soit par immersion dans une atmosphère de vapeur saturante.

Il faut toutefois noter que la méthode par trempage est délicate à mettre en oeuvre industriellement (sécurité, étape de séchage nécessaire...) et entraîne la mise en oeuvre d'une quantité importante de principes actifs, bien supérieure aux besoins de l'application et que, par ailleurs, la méthode par dispersion dans le copolymère n'est applicable qu'aux principes actifs thermostables.

Par contre, la méthode de chargement en substance active par diffusion à partir d'un support imprégné en substance active s'est révélée, de manière surprenante, particulièrement efficace et d'application industrielle aisée.

Selon un autre mode de mise en oeuvre dudit procédé, l'étape (d) de mise en forme est avantageusement réalisée par extrusion.

En variante, le procédé de préparation du film apte à servir de matrice de principe actif, conforme à l'invention, comprend :

(a) l'irradiation de l'EVA, sous forme de poudre, de granulé ou de film, à une dose comprise entre 10 et 80 KGy ;

(b) le stockage de l'EVA irradié pendant plusieurs semaines à température ambiante ou pendant plusieurs mois à basse température ;

(c) la copolymérisation EVA/polymère hydrophile par mise en contact de l'EVA irradié obtenu en (b) avec des monomères hydrophiles pendant plusieurs heures, de préférence pendant 5 à 20 h, pour l'obtention d'un copolymère EVA/polymère hydrophile, dans lequel le taux de greffage du polymère hydrophile est compris entre 10 et 50 % ;

(d) le chargement du copolymère en principe actif et s'il y a lieu, c'est-à-dire lorsque l'EVA est sous forme de poudre ou de granulé,

(e) la mise en forme du copolymère obtenu sous la forme d'un film.

La mesure du taux de greffage sur des échantillons tests doit être conforme à l'invention (10-50 %), après stockage.

Dans le cas où l'EVA est sous forme de film, à l'issue des étapes de greffages et de chargement en principe actif, un simple calandrage permettra d'amener ledit film à l'épaisseur désirée.

De tels procédés de préparation permettent :

- d'obtenir une grande variété de polymères, que l'on choisit, en fonction de la substance chimique ou principe actif à charger ; et
- d'éliminer le risque toxique lié à l'emploi de catalyseurs.

La présente invention a également pour objet un procédé de préparation d'un copolymère EVA/inclusions hydrophiles, tel que défini ci-dessus, caractérisé en ce qu'il comprend :

(a) l'irradiation de l'EVA, sous forme de poudre, de granulé ou de film, à une dose comprise entre 10 et 80 KGy ;
(b) le stockage de l'EVA irradié pendant plusieurs semaines à température ambiante ou pendant plusieurs mois à basse température ;
(c) la copolymérisation EVA/polymère hydrophile par mise en contact de l'EVA irradié obtenu en (b) avec des monomères hydrophiles pendant plusieurs heures, pour l'obtention d'un copolymère EVA/polymère hydrophile, dans lequel le taux de greffage du polymère hydrophile est compris entre 10 et 50 %.

Le temps de mise en contact EVA/monomères dépend de la dose absorbée, de la concentration en monomères et de la température ; elle est de préférence comprise entre 5 et 20 heures.

Selon un mode de mise en oeuvre avantageux dudit procédé, ledit copolymère obtenu est chargé en substance chimique, selon l'un des modes de chargement définis ci-dessus.

Un tel procédé permet de dissocier les étapes du procédé dans le temps, ce qui permet une meilleure adaptation à l'application industrielle et facilite le stockage de ces copolymères.

De tels copolymères sont particulièrement adaptés aux applications du domaine médical, agro-alimentaire ou agronomique, qui nécessitent la diffusion contrôlée d'une ou plusieurs substances chimiques, à partir d'un support solide pouvant être mis en forme (implant, par exemple, etc...).

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

**EXEMPLE 1 : Préparation d'un copolymère EVA 18/AA 26, apte à être utilisé comme film dans un système transdermique.**

**A. Méthode directe.**

**I - Composition du milieu réactionnel :**

On prépare une solution aqueuse contenant 18,56 kg d'acide acrylique, en présence de sel de Mohr puis, on ajoute 60 kg d'EVA 18, en poudre, à la solution ci-dessus.

Les caractéristiques principales de la solution obtenue sont :
volume total phase aqueuse $\neq$ 174,1

Rapport (masses) phase aqueuse/phase solide : 3,0
acide acrylique/EVA : 0,31

**II - Irradiation (atmosphère désoxygénée) :**

- Débit de dose : 600 Gy h$^{-1}$
- Dose absorbée : 15 KGy
- Durée : 24 h (fin de réaction déterminée par dosage de l'acidité résiduelle)

On obtient un taux de greffage en AA, de 26 % en 24 h environ.

Il faut noter que ces paramètres peuvent varier en fonction du dispositif d'irradiation utilisé.

**B. Méthode indirecte (méthode en 2 étapes).**

1) <u>Protocole opératoire</u> :

- Polymère support :

    . EVA à 18 %-22 %,

- Irradiation :

    . dose absorbée ≤ 40 KGy,
    . température ambiante.

- Conditions de conservation :

    . température ambiante (≈ 20°C)

- Greffage :

    . milieu réactionnel :

    monomère :                         acide acrylique qualité commerciale
    solvant :                          eau
    inhibiteur d'homopolymérisation :  sel de Mohr (C% = 8 g/l).

    . réacteur :

    nature verre
    volume nominal    1000 ml
    remplissage       { polymère : 150 g
                      { solution mère : 400<$V_{ml}$≤750

    . conditions opératoires :

    désaréation préalable :    flux d'azote pur
    température :              62,5 ± 0,5°C.

2) <u>Résultats (9 expériences)</u> :

Les Tableaux I et II suivants montrent le taux de greffage obtenu, en faisant varier les paramètres suivants : durée d'irradiation, durée de conservation, concentration en monomère et rapport pondéral monomère/EVA.

TABLEAU I

| N° expérience | Dose (KGy) | Durée conservation (jours) | Rapport AA/EVA | AA en sol. (g/l) |
|:---:|:---:|:---:|:---:|:---:|
| 1 | 20 | 14 | 0,4 | 150 |
| 2 | 40 | 14 | 0,4 | 120 |
| 3 | 20 | 4 | 0,4 | 120 |
| 4 | 40 | 4 | 0,4 | 150 |
| 5 | 20 | 14 | 0,6 | 150 |
| 6 | 40 | 14 | 0,6 | 120 |

TABLEAU I (suite)

| N° expérience | Dose (KGy) | Durée conservation (jours) | Rapport AA/EVA | AA en sol. (g/l) |
|---|---|---|---|---|
| 7 | 20 | 4 | 0,6 | 120 |
| 8 | 40 | 4 | 0,6 | 150 |
| 9 | 30 | 9 | 0,5 | 135 |

TABLEAU II

| REPONSES OBTENUES | | Expérience (N°) | | | | | | | | |
|---|---|---|---|---|---|---|---|---|---|---|
| | | 1 | 2 | 3 | 4 | 5 | 6 | 7 | 8 | 9 |
| Durée (h) | totale | 20 | 21h20 | 48 | 6h45 | 21h15 | 5h30 | 21h30 | 6h30 | 5h40 |
| | pour $\tau$ = 15% | 10-11 | 2h30 | 5 | 3 | 4h15 | 3 | 9 | 4 | 2h15 |
| | pour $\tau$ = 28% | 20 | 12 | 50 | 5h45 | 12 | 5h10 | 18 | 7 | 5 |
| Taux de greffage %, estimé d'après la | consommation en a. acrylique | 27,9 | 32,6 | 26,9 | 29,2 | 46,1 | 29,3 | 33,1 | 28,4 | 30,4 |
| | l'augmentation de masse | 28,9 | 32,9 | 22,3 | 29,9 | 47,4 | 30,7 | 33,3 | 27,1 | 28,4 |
| Rdt / A. Acrylique (%) | | 70 | 81 | 67 | 73 | (77) | 49 | 55 | 47 | 56 |

Il ressort de ces expériences que le taux de greffage de 26 % est atteint pour la quasi totalité des expériences. Ces résultats montrent également que cette méthode indirecte permet d'obtenir :

- une concordance satisfaisante entre les deux modes d'estimation des taux de greffage, à savoir par dosage de l'acide acrylique résiduel et d'après la variation de masse de polymère avant et après greffage. Ces résultats sont de surcroît corroborés par le dénombrement ultérieur des sites carboxyliques fixés dans le matériau obtenu,
- une durée de greffage comprise entre 5 et 20 h (n° 3 excepté),
- un rendement/acide acrylique : élevé (expériences 1, 2 et 4).

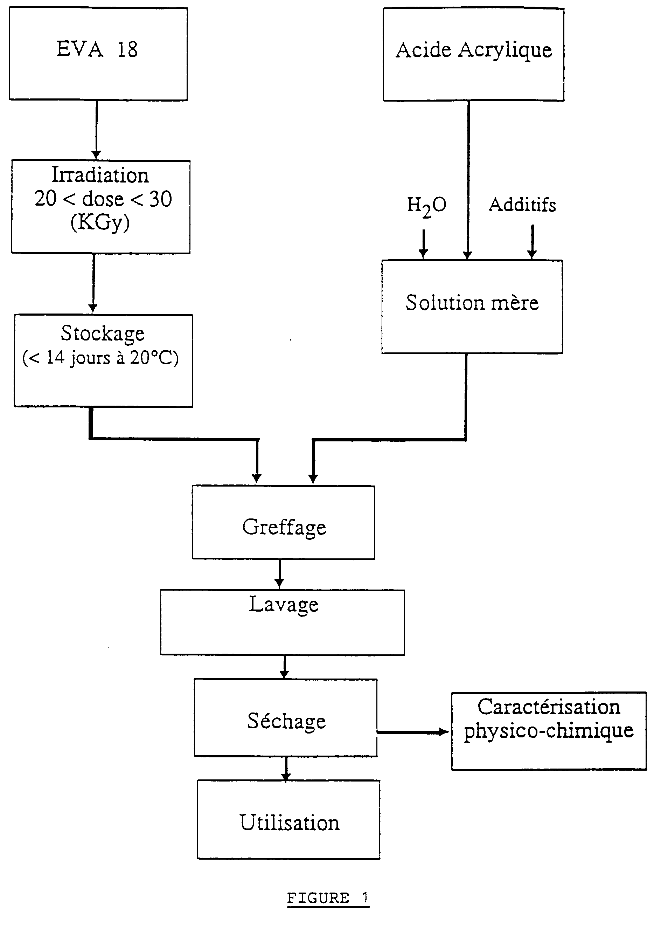

La figure 1 représente un schéma récapitulatif du protocole opératoire, selon cette méthode indirecte.

Aussi bien la méthode directe que la méthode indirecte, permettent d'obtenir un copolymère EVA/AA, dans lequel on a un pourcentage d'AA greffé, de l'ordre de 26 %. Ce copolymère peut se présenter sous forme de poudre, d'une granulométrie jusqu'à 500 µm, de granulé ou d'un film.

**EXEMPLE 2 : Préparation d'un film conforme à l'invention.**

A. **A partir d'un copolymère obtenu à l'exemple 1 :**

1) soit on réalise directement le film par extrusion à chaud (cas des principes actifs qui ne supportent pas la chaleur), puis on charge le film ainsi obtenu en principes actifs et en excipients (dont le stimulateur de perméabilité, si nécessaire), qui ne résistent pas à l'extrusion, à l'aide d'une des méthodes évoquées ci-dessus (trempage ou diffusion essentiellement) ;

2) soit on mélange à homogénéité le copolymère obtenu à l'exemple 1, avec le (les) principes actifs, les stimulateurs de perméabilité et les autres excipients résistant a l'extrusion (chargement par dispersion ou par immersion dans une atmosphère saturée), puis on transforme ledit mélange en granulés (diamètre : 2-3 mm ; longueur : 4-5 mm), et on extrude à chaud lesdits granulés, jusqu'à l'obtention d'un film, dans lequel d'autres excipients peuvent être rajoutés par trempage ou diffusion.

B. **Essais effectués sur le film obtenu :**

. contrôle de l'épaisseur du film,
. resistance à la déchirure (Norme NFQ 03-011),
. résistance à la traction (Norme NFT 54-102).

**EXEMPLE 3 : Préparation d'un film chargé en kétoprofène et système transdermique obtenu à partir de ce film.**

1) Obtention du copolymère EVA 18/AA 26 chargé en kétoprofène :

Le copolymère est obtenu conformément a l'exemple 1.

2) Préparation du mélange EVA/AA, kétoprofène, propylène glycol :

Le film comprenant du kétoprofène est préparé selon l'exemple 2, méthode A. 2).

Le propylène glycol (PG) et l'EVA-AA sont mis en contact durant 24 heures. Le kétoprofène est ensuite ajouté et le mélange est agité 30 minutes dans un mélangeur basculant.

* Granulation du mélange :

Le mélange est passé dans un malaxeur équipé de son système de découpe. Les granulés sont stockés dans des sacs en polyéthylène.

* Extrusion :

Le granulé EVA-AA-kétoprofène-propylène glycol est extrudé à l'aide d'une filière pour film plat. Un ensemble de traction-refroidissement est utilisé en sortie de filière.

Après extrusion, le film obtenu peut avoir une épaisseur contrôlée variant de 200 à 300 µm, suivant les besoins.

3) Mise en forme pharmaceutique : système transdermique :

On associe les différents composants dudit système transdermique par des méthodes connues ; on obtient alors un système transdermique comprenant de l'extérieur vers l'intérieur (contact avec la peau) :

- un film imperméable aluminisé souple (complexe aluminium/polyéthylène) (support) aux dimensions convenables.
- une couche de colle (XP 15362 B) préalablement diluée avec de l'acétate d'éthyle.
- le film de copolymère-principe actif.
- un film protecteur en polyester siliconé.

On enveloppe le système ainsi obtenu par exemple dans une pochette réalisée à partir d'une feuille aluminisée rigide (complexe aluminium/polyéthylène) soudée sur une feuille de film aluminisée rigide à double face (argent/blanc).

A. **Etude de désorption et de la dose de charge en principe actif du film obtenu.**

* Test de désorption :

- Film utilisé :

. polymère : EVA 18/AA 26
. charge du kétoprofène : 10 %
  du PG : 25 %,
. épaisseur mm : 0,30,
. surface cm$^2$ : 7,06.

- Conditions opératoires :

. appareil de dissolution : Dissolutest$^®$ (USP XX) PROLABO,
. milieu de désorption : tampon phosphate 0,05 M,

. pH : 6,5,
. volume : 113 ml/cm$^2$,
. température : 32°C,
. essai réalisé à volume constant : 800 ml.

- Méthode analytique :

. mesure de la DO en spectrophométrie UV à λ max : 260 nm,
. la concentration est calculée par référence à une gamme d'étalonnage.

* Résultats :

Moyennes et écart-types de 6 expériences (Tableau III).

TABLEAU III

| Période heures | Quantité totale désorbée mg | Rendement de la désorption % | Taux moyen de désorption mg/24h/cm$^2$ |
|---|---|---|---|
| 0 | 0,0 ± 0,0 | 0,0 ± 0,0 | |
| 0,08 | 6,6 ± 0,4 | 33,2 ± 2,0 | |
| 0,17 | 8,0 ± 0,4 | 40,1 ± 2,1 | |
| 0,25 | 8,3 ± 0,5 | 41,8 ± 2,4 | |
| 0,33 | 8,4 ± 0,5 | 42,3 ± 2,4 | |
| 0,5 | 8,7 ± 0,5 | 43,8 ± 2,6 | |
| 0,67 | 9,0 ± 0,5 | 45,3 ± 2,4 | |
| 1 | 9,4 ± 0,5 | 47,4 ± 2,7 | |
| 1,5 | 9,9 ± 0,6 | 49,9 ± 2,9 | |
| 2 | 10,4 ± 0,6 | 52,2 ± 2,8 | |
| 4 | 11,8 ± 0,6 | 59,3 ± 2,8 | |
| 6 | 13,1 ± 0,7 | 65,7 ± 3,6 | |
| 8 | 14,1 ± 0,9 | 71,0 ± 4,5 | |
| 24 | 18,5 ± 1,1 | 92,7 ± 5,5 | 2,61 |
| 48 | 19,9 ± 1,1 | 99,8 ± 5,5 | |

**B. Contrôle du produit fini : désorption du système transdermique à base de kétoprofène.**

Le contrôle de la charge en principe actif est effectué comme ci-dessus, en A.

* Résultats :

Moyennes et écart-types de 6 expériences (Tableau IV).

TABLEAU IV

| Période heures | Quantité totale désorbée mg | Rendement de la désorption % | Taux moyen de désorption mg/24h/cm$^2$ |
|---|---|---|---|
| 0 | 0,0 ± 0,0 | 0,0 ± 0,0 | |
| 0,08 | 1,6 ± 0,5 | 7,6 ± 2,6 | |

TABLEAU IV (suite)

| Période heures | Quantité totale désorbée mg | Rendement de la désorption % | Taux moyen de désorption mg/24h/cm$^2$ |
|---|---|---|---|
| 0,17 | 2,1 ± 0,7 | 10,1 ± 3,4 | |
| 0,25 | 2,4 ± 0,9 | 11,8 ± 4,2 | |
| 0,33 | 2,7 ± 1,0 | 13,2 ± 4,7 | |
| 0,5 | 3,2 ± 1,1 | 15,5 ± 5,5 | |
| 0,67 | 3,6 ± 1,3 | 17,3 ± 6,2 | |
| 1 | 4,2 ± 1,5 | 20,7 ± 7,2 | |
| 1,5 | 5,0 ± 1,7 | 24,3 ± 8,5 | |
| 2 | 5,6 ± 1,9 | 27,1 ± 9,3 | |
| 4 | 7,1 ± 2,3 | 34,8 ± 11,3 | |
| 6 | 8,1 ± 2,4 | 39,6 ± 11,7 | |
| 8 | 8,9 ± 2,4 | 43,4 ± 11,9 | |
| 24 | 13,7 ± 2,6 | 66,7 ± 12,5 | 1,03 |
| 48 | 16,4 ± 1,7 | 80,2 ± 8,3 | |

**EXEMPLE 4 : Préparation d'un système transdermique selon l'invention (principe actif : trinitrine).**

On procède d'une manière analogue à celle des exemples 1 et 2, avec comme polymère hydrophobe de départ, de l'EVA 33.
On obtient alors un copolymère EVA 33/AA 26.

A. **Etude de désorption et de la dose de charge en principe actif du film.**

* Test de désorption :

- Film utilisé :

   . polymère : EVA 33/AA 26,
   . charge en % du PA : 17
   . épaisseur mm : 0,30,
   . surface cm$^2$ : 7,06.

- Conditions opératoires :

   . Appareil de dissolution : Dissolutest® (USP XX) PROLABO,
   . milieu de désorption : eau désionisée,
   . pH : 4,5-5,
   . volume/unité de surface du film : 127 ml/cm$^2$
   . température : 32°C,
   . essai réalisé à volume constant : 900 ml.

- Méthodes analytiques :

   . chromatographie phase gazeuse,
   . la concentration est calculée par référence à une gamme d'étalonnage.

* Résultats :

TABLEAU V

| Période heures | Quantité totale désorbée mg | Rendement de la désorption % | Taux moyen de désorption mg/24h/cm$^2$ |
|---|---|---|---|
| 0,08 | 2,0 ± 0,4 | 5,6 ± 1,0 | |
| 0,17 | 3,0 ± 0,4 | 8,5 ± 0,9 | |
| 0,25 | 3,7 ± 0,4 | 10,7 ± 1,0 | |
| 0,33 | 4,5 ± 0,4 | 12,8 ± 1,1 | |
| 0,5 | 5,6 ± 0,8 | 16,0 ± 2,3 | |
| 0,67 | 6,7 ± 0,6 | 19,1 ± 1,8 | |
| 1 | 8,6 ± 0,4 | 24,5 ± 1,2 | |
| 1,5 | 10,6 ± 0,9 | 30,4 ± 2,1 | |
| 2 | 12,8 ± 1,1 | 36,5 ± 3,1 | |
| 4 | 20,5 ± 2,0 | 58,6 ± 5,4 | |
| 6 | 26,6 ± 2,2 | 76,2 ± 6,4 | |
| 8 | 28,1 ± 1,4 | 80,5 ± 3,7 | |
| 24 | 37,5 ± 1,3 | 107,3 ± 5,0 | 5,31 |

**B. Contrôle du produit fini : désorption d'un système transdermique à base de trinitrine préparé conformément à l'Exemple 2.**

Le contrôle de la charge en principe actif est effectué comme ci-dessus en A.

* Résultats :

Moyennes et écart-types de 5 expériences (Tableau VI).

TABLEAU VI

| Période heures | Quantité totale désorbée mg | Rendement de la désorption % | Taux moyen de désorption mg/24h/cm$^2$ |
|---|---|---|---|
| 0 | 0,0 ± 0,0 | 0,0 ± 0,0 | |
| 0,08 | 1,0 ± 0,3 | 3,6 ± 1,2 | |
| 0,17 | 1,7 ± 0,5 | 6,5 ± 1,9 | |
| 0,25 | 2,2 ± 0,6 | 8,2 ± 2,2 | |
| 0,33 | 2,8 ± 0,9 | 10,7 ± 3,3 | |
| 0,5 | 4,7 ± 0,2 | 18,0 ± 0,9 | |
| 0,67 | 5,4 ± 0,5 | 20,5 ± 2,0 | |
| 1 | 6,7 ± 0,6 | 25,5 ± 2,2 | |
| 1,5 | 7,9 ± 0,5 | 30,0 ± 1,8 | |
| 2 | 9,1 ± 0,4 | 34,4 ± 1,4 | |
| 4 | 12,0 ± 2,0 | 45,3 ± 7,6 | |
| 6 | 14,7 ± 1,7 | 55,8 ± 6,3 | |

TABLEAU VI (suite)

| Période heures | Quantité totale désorbée mg | Rendement de la désorption % | Taux moyen de désorption mg/24h/cm$^2$ |
|---|---|---|---|
| 8 | 16,9 ± 1,1 | 63,9 ± 4,1 | |
| 24 | 21,3 ± 2,3 | 80,7 ± 8,6 | 3,02 |
| 48 | 25,6 ± 4,4 | 96,9 ± 16,7 | |

**EXEMPLE 5 : Comparaison (cinétique de l'absorption ou de désorption) de l'EVA 18/AA 26 et de l'EVA 18/AA 33 : exemple de la nicotine.**

**I - Absorption de nicotine par des films irradiés :**

A. **Réticulation des films :**

1. Nature des films :

- films obtenus par extrusion de poudres polymères :

    a) EVA 33/AA 26
    b) EVA 18/AA 26
    c) EVA 33

- épaisseur = 0,3 mm et 40 cm environ de long sont traités.

2. Protocole :

Les films (40 cm) sont placés dans des sachets de polyéthylène purgés et remplis d'azote avant d'être fermés par soudure.

B. **Absorption sur films :**

1. Protocole :

- solution à 2 % environ en nicotine (p/v) dans l'eau, 46 ml pour un échantillon de 24 mm de diamètre, et de surface d'échange égale à 9,3 cm$^2$.

On prépare un film comme précisé à l'exemple 2, méthode A. 1) (trempage) : l'absorption est faite pendant, d'une part, 3 heures et, d'autre part, 24 heures à 37°C dans un bain-marie à agitation latérale.

En fin d'absorption les échantillons sont essuyés. Ils ne peuvent être séchés de façon habituelle en étuve, la nicotine étant très volatile.

2. Résultats :

Les taux d'absorption sont calculés par prise de poids des échantillons en référence à un témoin qui a subi le même traitement dans l'eau. Ils sont calculés par rapport au poids final.

Les résultats sont rassemblés dans le Tableau VII.

TABLEAU VII

| Absorption de nicotine par différents films | | | |
|---|---|---|---|
| Films | Pf (g) | P (mg) | Absorption (mg/g) |
| Absorption pendant 3 heures - Nicotine à 2 % | | | |
| EVA 18/AA 26 | 0,1920 | 39 | 202 |
| EVA 33/AA 26 | 0,2132 | 37,6 | 176 |
| EVA 33 | 0,1704 | 1,4 | 8,2 |
| Absorption pendant 24 heures - Nicotine à 2 % | | | |
| EVA 18/AA 26 | 0,1842 | 28,6 | 155,3 |
| EVA 33/AA 26 | 0,2591 | 79,5 | 306,8 |
| EVA 33 | 0,1709 | 2,4 | 14,0 |
| Pf : poids du film ; P : poids de nicotine. | | | |

## II - Désorption *in vitro* de nicotine par les films obtenus en I.

### A. Désorption après une absorption de trois heures :

1. Protocole :

  . films : EVA 18/AA 26
    EVA 33/AA 26 :
    e = 0,3 mm

  . volume de désorption : 46 ml d'eau par échantillon de 24 mm de diamètre et de 9,3 cm$^2$ de surface d'échange. Maintien de la température à 37°C dans un bain-marie à agitation latérale.

  Renouvellement du milieu toutes les 2 heures.

2. Résultats :

  Ils sont rassemblés dans les Tableaux VIII et IX.

TABLEAU VIII

| Désorption des films d'EVA 18/AA 26 (absorption de nicotine pendant 3 h) | | | |
|---|---|---|---|
| Temps (heure) | Désorption | | Flux (µg/h/cm$^2$) |
| | (mg) | (cumul) | (%) | |
| 2 | 5,05 | 5,05 | 13 | 272 |
| 4 | 3,25 | 8,30 | 21 | 173,5 |
| 6 | 1,40 | 9,70 | 25 | 76,5 |
| 8 | 1,65 | 11,35 | 29 | 90 |
| 10 | 1,30 | 12,65 | 32 | 69,5 |
| 12 | 1,15 | 13,75 | 35 | 61 |
| 14 | 1,10 | 14,85 | 38 | 57,5 |

TABLEAU IX

| Désorption des films d'EVA 33/AA 26 (absorption de nicotine pendant 3 h) | | | | |
|---|---|---|---|---|
| Temps (heure) | Désorption | | | Flux ($\mu g/h/cm^2$) |
| | (mg) | (cumul) | (%) | |
| 2 | 3,45 | 3,45 | 9 | 185 |
| 4 | 2,10 | 5,55 | 15 | 112,5 |
| 6 | 1,70 | 7,25 | 19 | 90,5 |
| 8 | 1,70 | 8,95 | 24 | 91 |
| 10 | 1,05 | 10 | 27 | 56,5 |
| 12 | 1,9 | 11,9 | 32 | 102 |
| 14 | 1,3 | 13,2 | 35 | 69,5 |

B. **Désorption après absorption de 24 heures :**

1. Protocole :

. Films : EVA 18/AA 26
EVA 33/AA 26
EVA 33
e = 0,3 mm, s = 9,3 $cm^2$
. volume de désorption : 46 ml d'eau par échantillon, maintien de la température à 37°C dans un bain-marie à agitation latérale.
. Renouvellement du milieu toutes les 2 heures.

2. Résultats :

Ils sont rassemblés dans les Tableaux X, XI, XII.

TABLEAU X

| Désorption de nicotine à partir de films d'EVA 33 (absorption pendant 24 h) | | | | |
|---|---|---|---|---|
| Temps (heure) | Désorption | | | Flux ($\mu g/h/cm^2$) |
| | (mg) | (cumul) | (%) | |
| 2 | 1,25 | 1,25 | 52 | 67 |
| 4 | 0,10 | 1,35 | 56 | 4,5 |
| 6 | 0,02 | 1,37 | 57 | 1 |
| 8 | 0,01 | 1,38 | 57,5 | 0,5 |
| 10 | 0,01 | 1,39 | 58 | 0,5 |
| 12 | 0,01 | 1,40 | 58 | 0,5 |
| 14 | 0,01 | 1,41 | 59 | 0,5 |

TABLEAU XI

| Désorption de nicotine à partir de films d'EVA 18/AA 26 (absorption pendant 24 h) | | | | |
|---|---|---|---|---|
| Temps (heure) | Désorption | | | Flux ($\mu$g/h/cm$^2$) |
| | (mg) | (cumul) | (%) | |
| 2 | 7,1 | 7,1 | 25 | 383,5 |
| 4 | 3,4 | 10,5 | 37 | 182 |
| 6 | 2,5 | 13 | 46 | 135 |
| 8 | 1,2 | 14,2 | 50 | 68 |
| 10 | 1 | 15,2 | 53 | 54 |
| 12 | 0,8 | 16 | 56 | 43 |
| 14 | 0,7 | 16,7 | 59 | 37 |

TABLEAU XII

| Désorption de nicotine à partir de films EVA 33/AA 26 (absorption pendant 24 h) | | | | |
|---|---|---|---|---|
| Temps (heure) | Désorption | | | Flux ($\mu$g/h/cm$^2$) |
| | (mg) | (cumul) | (%) | |
| 2 | 19,6 | 19,6 | 25 | 1055 |
| 4 | 6 | 25,6 | 32 | 325 |
| 6 | 4,1 | 29,7 | 37 | 250 |
| 8 | 2,5 | 32,2 | 40 | 135 |
| 10 | 1,9 | 34,1 | 43 | 103 |
| 12 | 1,8 | 35,9 | 45 | 95 |
| 14 | 1,7 | 37,6 | 47 | 90 |

Ces données montrent notamment que l'EVA 18/AA 26 :

- permet d'atteindre la charge maximale d'absorption rapidement (voir Tableau VII) ;
- a une vitesse de désorption rapide.

**EXEMPLE 6** : Comparaison du comportement de l'EVA 18/AA 26 et de l'EVA 18/AA 16 : exemple de la nicotine.

TABLEAU XIII

| Tableau synoptique des principaux résultats issus de l'analyse physico-chimique des supports. | | | |
|---|---|---|---|
| Critère de comparaison | | Type de copolymère | |
| | | EVA 18/AA 16 | EVA 18/AA 26 |
| Composition centésimale % | C | 68,7 ± 0,1 | 71,5 ± 0,3 |
| | H | 10,9 ± 0,1 | 11,4 ± 0,1 |
| | O | 18,2 ± 0,1 | 17,0 ± 0,1 |
| | N | inf. à 1 % | inf. à 1 % |
| Capacité d'échange | $(mEq.g^{-1})$ | 1,91 ± 0,11 | 2,87 ± 0,04 |
| Taux de greffage estimé | (%) | 15,9 ± 0,9 | 26,0 ± 0,4 |
| Perte de masse (%) à l'analyse thermogravimétrique | 150°C | 1,3 | 0,6 |
| | 250°C | 3,3 | 1,9 |
| | 400°C | 25,3 | 22,1 |
| | 500°C | 91,7 | 90,8 |
| Spectrométrie à l'infrarouge | $CH_2$ | + | + |
| | C=O ester | + | + |
| | C-O-C | + | + |
| | C=O acide | + | + |
| | OH acide | + | + |
| Spectrométrie de masse (m/e) | 72 | + | + |
| | 86 | + | + |
| | 99 | + | + |
| | 13 | + | + |
| Fraction soluble dans l'eau | (%) | 4,2 | 2,8 |
| Fraction soluble dans le toluène | (%) | 62 | 51 |

TABLEAU XIV

| Tableau synoptique des principaux résultats issus de l'étude de comportement des supports. | | | |
|---|---|---|---|
| Critère de comparaison | | Type de copolymère | |
| | | EVA18/AA16 | EVA18/AA26 |
| Durée de chargement des poudres | (min) | 15 | 15 |
| Taux de chargement des poudres | (%) | 35 | 25 |
| Quantité d'eau après chargement | (%) | 379 | 148 |
| Cinétique de désorption des poudres | (mg/45 min) | 78 | 74 |
| Taux de chargement des films | $(mg.cm^{-2})$ | 7,78±0,56 | 6,40±0,30 |
| Augmentation de surface après imprégnation | | 2,6 | 1,9 |
| Augmentation de surface après séchage | | 1,4 | 1,3 |
| Augmentation de poids après imprégnation | | 4,7 | 2,9 |
| Augmentation de poids après séchage | | 2,0 | 1,8 |
| Quantité d'eau après chargement | $(mg.cm^{-2})$ | 24,1 | 17,1 |
| Quantité d'eau après séchage | $(mg.cm^{-2})$ | 6,6 | 7,7 |
| Cinétique de désorption des films | | | |
| - demi-vie de la première phase | (heure) | 0,63 | 0,81 |
| - demi-vie de la dernière phase | (heure) | 20,1 | 20,1 |

Ces données montrent également l'intérêt du copolymère EVA 18/AA 26. La quantité d'eau adsorbée au cours du chargement est très nettement augmentée dans le cas de l'utilisation de l'EVA 18/AA 26 ; ceci entraîne un gonflement important du film et altère ses propriétés mécaniques.

**EXEMPLE 7 : Préparation d'un film chargé en physostigmine et système transdermique obtenu à partir de ce film.**

1) <u>Obtention du copolymère EVA 18/AA 26 chargé en physostigmine à 5 mg/cm$^2$</u> :

Le copolymère est obtenu conformément à l'exemple 1.

2) <u>Préparation du mélange EVA/AA, physostigmine</u> :

Le film comprenant de la physostigmine est préparé selon l'Exemple 2, méthode A. 1) :
l'incorporation de la physostigmine est réalisée par imprégnation du film dans une solution éthanolique de physostigmine.
Les conditions de chargement sont les suivantes :

- composition de la solution $H_2O$/EtOH : 80/20 (v/v) ;
- concentration de la solution en physostigmine : 20 mg/ml ;
- conditions de trempage : à l'abri de la lumière et sous agitation pendant 3 heures.

Un tel temps de chargement permet d'obtenir un taux de charge autour de 5 mg/cm$^2$.

3) <u>Mise en forme pharmaceutique : système transdermique</u> :

On associe les différents composants dudit système transdermique par des méthodes connues ; on obtient alors un système transdermique comprenant de l'extérieur vers l'intérieur (contact avec la peau) les mêmes constituants qu'à l'exemple 3.

A. **Etude de la désorption et de la dose de charge en principe actif du film obtenu.**

\* <u>Test de désorption</u> :

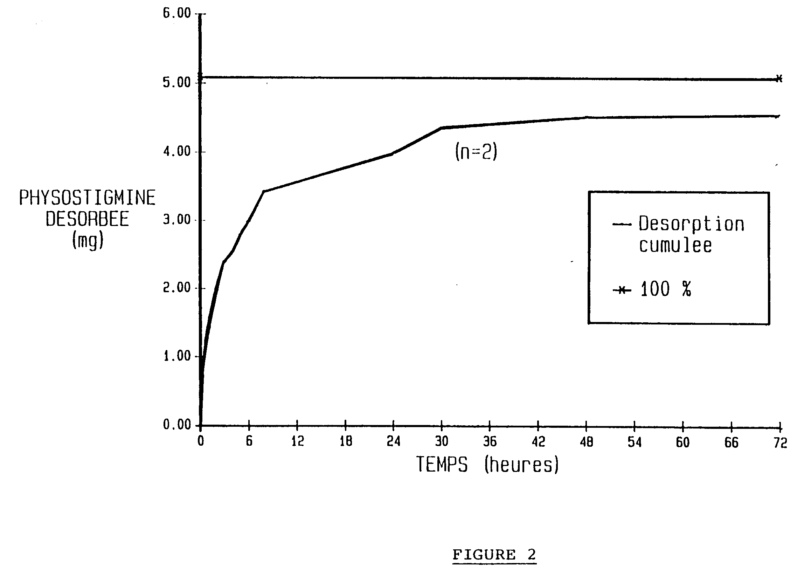

Des profils de désorption ont été réalisés, dans l'eau à 37°C, sur des pastilles de film chargées dans les conditions précédentes, 3 jours et 38 jours après chargement. Ces profils sont représentés figure 2 (abscisse : temps (heures), ordonnée : physostigmine chargée (mg/cm$^2$)).

On observe une désorption progressive de la physostigmine libérée par le film.

50 % de ce qui a été fixé est désorbé en trois heures (T50) et plus de 90 % au bout de 72 heures (T90). La fraction non libérable est faible (moins de 10 %). Les deux profils sont pratiquement similaires indiquant une stabilité des conditions de désorption au cours du temps écoulé (38 j).

B. **Contrôle du produit fini : désorption du système transdermique à base de physostigmine.**

Le contrôle de charge est réalisé en dosage en HPLC, précédé d'une extraction de la physostigmine du film.

La stabilité du taux de charge a été vérifié sur six semaines. Il n'a pas été mis en évidence de variation du taux de charge au cours du temps, ce taux étant constant autour de 5 mg/cm$^2$.

C. **Etude pharmacocinétique de la libération du principe actif à partir dudit système transdermique.**

Les études pharmacocinétiques et pharmacologiques menées chez le lapin, au cours de l'application de systèmes transdermiques de 50, 20 et 12 mm de diamètre (19,63, 3,14 et 1,13 cm$^2$ de surface), ont montré que les concentrations plasmatiques de physostigmine se maintiennent à un plateau pendant toute la durée de l'application, et son activité par la modification de l'activité cholinestérasique.

Le système transdermique délivre la physostigmine avec un flux moyen efficace de 6,29 ± 2,6 µg/h.cm$^2$.

La demi-vie de la physostigmine après retrait du patch est équivalente à la demi-vie après administration par voie intraveineuse de physostigmine, et le dosage de la physostigmine au niveau cutané révèle des taux négligeables de physostigmine. Il n'existe donc pas de phénomène réservoir au niveau de la peau.

<u>EXEMPLE 8</u> : **Préparation d'un film chargé en 17β estradiol et système transdermique obtenu à partir de ce film.**

1) <u>Obtention du copolymère EVA 18/AA 26 chargé en 17β estradiol à 7 %</u> :

Le copolymère est obtenu conformément a l'exemple 1.

2) <u>Préparation du mélange EVA/AA. 17β estradiol, propylène glycol, polyéthylène glycol 400</u> :

Le film comprenant du 17β estradiol est préparé selon l'Exemple 2, méthode A. 2) :

le propylène glycol, le PEG 400 et l'EVA/AA sont mis en contact pendant 24 heures, puis le 17β estradiol est ajouté et le mélange est agité 30 min dans un mélangeur basculant.

On procède ensuite comme dans l'exemple 3.

Lorsque le film est réalisé, il est chargé en éthanol soit par immersion dans une atmosphère saturée en éthanol (exemple 2, méthode A.2), soit par diffusion à partir d'un support imprégné en éthanol (exemple 2, méthode A.1), pour augmenter le passage cutané de l'estradiol.

3) <u>Mise en forme pharmaceutique : système transdermique</u> :

On associe les différents composants dudit système transdermique par des méthodes connues ; on obtient alors un système transdermique comprenant de l'extérieur vers l'intérieur (contact avec la peau) :

- un film imperméable aluminisé souple (complexe aluminium/polyéthylène) (support) aux dimensions convenables : 40 cm$^2$.
- une couche de colle (XP 15362 B : copolymère acrylique) préalablement diluée avec de l'acétate d'éthyle : 80 mg.
- le film de copolymère-principe actif : 1200 mg, avec PEG 400 60 mg et PG 300 mg.
- un film protecteur en polyester fluoré : 40 cm$^2$.

On enveloppe le système ainsi obtenu par exemple dans une pochette réalisée à partir d'une feuille aluminisée rigide (complexe aluminium/polyéthylène) soudée sur une feuille de film aluminisée rigide à double face (argent/blanc).

Les différents composants du système transdermique sont soumis aux mêmes contrôles *in vitro* que précédemment (voir exemple 3).

Les études pharmacocinétiques menées chez des femmes ménopausées, au cours de l'application de systèmes transdermiques de 40 cm$^2$, ont montré que les concentrations plasmatiques de 17β estradiol restent égales aux concentrations reconnues efficaces pendant au moins 4 jours. La concentration moyenne est de 34 ± 7 pg/ml entre 0 et 96 heures, comme le montre les résultats suivants :

des prélèvements effectués avant pose du système transdermique conforme à l'invention (t = -24 h), donnent le taux de base de 17β estradiol et est de l'ordre de 6 ± 1 pg/ml.

Le Tableau XV ci-après présente les valeurs moyennes des concentrations plasmatiques du passage transcutané de l'estradiol après application d'un système transdermique conforme à l'invention, durant 96 h (n = 4).

TABLEAU XV

| Temps heures | concentrations plasmatiques moyennes ± écart type |
|---|---|
| -24 | 6 ± 1 |
| 0 | 15 ± 12 |
| 5 | 55 ± 16 |
| 9 | 68 ± 28 |
| 24 | 47 ± 10 |
| 29 | 34 ± 12 |
| 33 | 35 ± 18 |
| 48 | 29 ± 17 |
| 72 | 28 ± 16 |
| 96 | 25 ± 8 |
| 120 | 8 ± 2 |

**<u>EXEMPLE 9</u> : Chargement d'une matrice en éthanol par diffusion à partir d'un support (non tissé) imprégné en principe actif (17β estradiol).**

TABLEAU XVI

| Durée du transfert | Teneur en alcool moyen (n=2) | |
|---|---|---|
| | mg | % |
| 1,5 | 6,9 | 21 |
| 4 | 8,8 | 28 |
| 6 | 9,1 | 27 |
| 24 | 9,0 | 27 |
| 48 | 9,7 | 29 |

Ce Tableau XVI présente les résultats du chargement en éthanol d'un film chargé en 17β estradiol conforme à l'exemple 8, par diffusion à partir d'un support non tissé imprégné en éthanol.

Ceci illustre que dès 1 h 30, on obtient des taux de chargement en éthanol suffisants pour l'application transdermique.

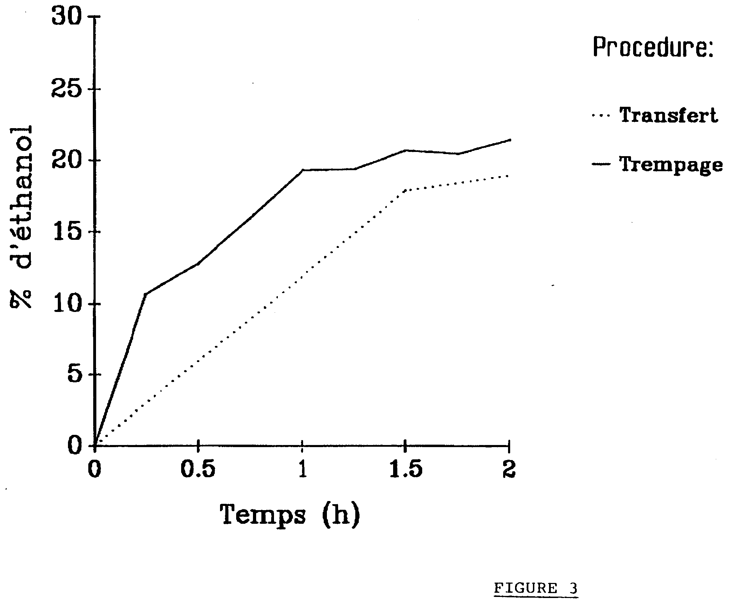

La figure 3 compare ce nouveau mode de chargement (diffusion) avec le chargement par trempage dont les inconvénients ont été rappelés plus haut, et montre l'équivalence de ces procédés de chargement.

**EXEMPLE 10 : Préparation d'un copolymère EVA/AAm, apte à être utilisé comme film dans un système transdermique.**

1) Protocole opératoire :

La préparation des solutions, le dégazage, le greffage, les lavages et le séchage sont réalisés comme précisé à l'Exemple 1, avec comme monomère hydrophile, de l'acrylamide.

En particulier, l'irradiation de l'EVA 20 (EXXON) est réalisée à une dose comprise entre 20 et 40 kGy et est suivie d'un vieillissement à température ambiante compris entre 4 et 10 jours ; le rapport massique acrylamide (AAm)/EVA est compris entre 0,4 et 0,6 et la concentration de l'acrylamide en solution aqueuse de l'ordre de 150 g.l$^{-1}$.

De manière plus précise, le protocole comprend :

a) l'irradiation de l'échantillon d'une dose de 40 kGy sous faisceau d'électrons de 17 MeV, issus d'un accélérateur linéaire et caractérisé par un débit de dose de l'ordre de $1,5 \times 10^6$ Gy.h$^{-1}$, selon les conditions opératoires adoptées.

b) le chauffage à l'abri de l'air, pendant 7 h à 60°C dans une solution à 20 % en acrylamide, dans de l'eau contenant 4 g.l$^{-1}$ de sel de Mohr.

Après lavage et séchage, on trouve par gravimétrie, un taux de greffage égal à 25 %, la cinétique de greffage étant suivie par réfractométrie ou spectrophotométrie UV sur des prélèvements intermittents de la solution de greffage.

2) Résultats (4 essais) :

Le Tableau XVII montre le taux de greffage obtenu, en faisant varier les paramètres suivants : durée de l'irradiation, durée de conservation (vieillissement), concentration en monomère et rapport pondéral monomère/EVA.

TABLEAU XVII

| Essai N° | Dose absorbée (kGy) | Durée de vieillisst (jours) | AAm/EVA | Durée de réaction (min) | Durée τ = 15% (min) | Durée τ = 28% (min) | τ estimé par UV (%) | τ pondéral (%) |
|---|---|---|---|---|---|---|---|---|
| 1 | 30 | 9 | 0.5 | 360 | 230 | 375 | 27.3 | 33.0 |
| 2 | 40 | 7 | 0.6 | 285 | 100 | 290 | 27.8 | 26.8 |
| 3 | 20 | 4 | 0.6 | 375 | - | - | | 18.3 |
| 4 | 40 | 7 | 0.4 | 375 | 290 | 530 | 19.5 | 21.6 |

Il ressort de ces essais que le taux de greffage de 26 % est atteint pour la quasi totalité des expériences ; on constate une assez bonne adéquation entre les valeurs du taux de greffage estimées par spectrophotométrie UV et pondérales.

Ces résultats montrent également que :

- la vitesse de greffage est d'autant plus grande que la dose absorbée est importante (essais 2 et 3),
- la quantité d'acrylamide rapportée à l'EVA est élevée (essais 2 et 4),
- l'essai AAm n° 1 présente des paramètres cinétiques proches de ceux de l'essai n° 9 de greffage de l'acide acrylique, effectué dans les mêmes conditions,
- les meilleurs résultats obtenus avec l'acrylamide (essai AAm n° 2) sont également comparables à ceux obtenus avec l'acide acrylique (essai n° 4).

3) Caractérisation des copolymères obtenus :

Comme dans le cas de l'acide acrylique, les EVA greffés à l'acrylamide ont été caractérisés par différentes techniques afin, d'une part, de vérifier leur structure (nature des greffons, taux de greffage...), et surtout de vérifier s'ils possédaient les propriétés hydrophiles espérées.

a) **Analyse élémentaire :**

Cette technique, non adaptée aux EVA greffés à l'acide acrylique, retrouve son intérêt, du fait de l'introduction d'un hétéroatome, en l'occurrence l'azote. La teneur de cet élément, qui n'est pas présent dans l'EVA de départ, est susceptible de varier de façon suffisamment significative pour pouvoir être corrélée sans trop d'erreur avec le taux de greffage en acrylamide.

Ainsi, si l'on appelle x, y et z les fractions pondérales respectives en éthylène, acétate de vinyle et acrylamide dans le polymère greffé (sur la base de $x + y = 100\%$ et $z = \tau$), on obtient :

$$\%N = \frac{19.774z}{(x + y + z)} \quad \text{soit} \quad \tau = \frac{100. \%N}{19.774 - \%N}$$

La comparaison des différentes valeurs du taux de greffage (valeurs estimées, pondérales et recalculées d'après la teneur en azote) laisse apparaître une grande homogénéité. D'une part, pour un même échantillon, à une exception près, la teneur en azote varie peu d'une prise d'essai à l'autre ce qui témoigne d'un greffage homogène. D'autre part, les taux de greffage recalculés concordent très bien avec les autres valeurs et confirment ainsi la validité du suivi cinétique par UV.

TABLEAU XVIII

| Essai N° | % C | % H | % N | Taux de greffage (%) | | |
|---|---|---|---|---|---|---|
| | | | | recalculé | d'après % N | pondéral |
| 1 | 70.14 | 11.66 | 5.02 | 34.0 | 30.6 ± 2.4 | 33.0 |
| | 70.25 | 11.90 | 4.40 | 28.6 | | |
| | 69.63 | 11.27 | 4.47 | 29.2 | | |
| 2 | 71.32 | 11.59 | 3.89 | 24.5 | 24.6 ± 1.3 | 26.8 |
| | 72.46 | 11.75 | 3.71 | 23.1 | | |
| | 70.76 | 11.20 | 4.12 | 26.3 | | |
| 3 | 74.18 | 11.64 | 2.95 | 17.5 | 17.7 ± 0.2 | 18.3 |
| | 73.59 | 11.64 | 3.01 | 18.0 | | |
| | 73.69 | 11.42 | 2.96 | 17.6 | | |
| 4 | 73.44 | 11.18 | 3.43 | 21.0 | 21.5 ± 0.5 | 21.6 |
| | 72.79 | 11.36 | 3.56 | 22.0 | | |
| | 72.26 | 11.22 | (6.57) | (49.8) | | |

b) **Reprise en humidité :**

L'hydrophilie des polymères greffés a été évaluée d'après leur reprise en humidité, Wm (exprimée en ‰ en masse). Cette caractéristique de chaque matériau est reliée à sa structure par une relation d'additivité qui, dans le cas du greffage de l'acrylamide, s'exprime selon la relation (1) :

$$Wm\ (\%_o) = \frac{18\ 000}{100 + \tau} \left[ \frac{VA}{86}.Hi\ (ester) + \frac{\tau}{71}.Hi\ (amide) \right]$$

où les Hi représentent les contributions élémentaires des maillons hydrophiles. Celles-ci sont fonction du taux d'hygrométrie et se calculent par interpolation des valeurs de Van Krevelen :

TABLEAU XIX

| Humidité relative, HR (%) | 44 | 65 | 81 | 97 |
|---|---|---|---|---|
| Hi ($CO_2CH_3$) | 0.04 | 0.07 | 0.11 | 0.19 |
| Hi ($CONH_2$) | 0.45 | 0.69 | 1.16 | 1.85 |

Le Tableau suivant présente une synthèse des résultats expérimentaux (première valeur) et des valeurs recalculées d'après la relation (1) avec le taux de greffage pondéral (première parenthèse) ou le taux d'après la teneur en azote (seconde parenthèse) :

TABLEAU XVII

| | Taux de greffage (%) | | Reprise en humidité à l'équilibre, Wm (‰) | | | |
|---|---|---|---|---|---|---|
| Essai N° | pondéral | % N | HR = 44% | HR = 65% | HR = 81% | HR = 97% |
| 1 | 33.0 | 30.6 | 23 (30) (28) | 42 (46) (43) | 62 (76) (72) | 174(122)(116) |
| 2 | 26.8 | 24.6 | 16 (26) (24) | 30 (39) (37) | 51 (66) (62) | 121 (105) (99) |
| 4 | 21.6 | 21.5 | 15 (22) (22) | 26 (33) (33) | 46 (56) (56) | 123 (90) (90) |

Comme dans l'Exemple 1 (EVA greffés à l'acide acrylique), la reprise en humidité croit avec le taux d'hygrométrie (HR) et, pour un taux d'hygrométrie donné, avec le taux de greffage.

Si l'on compare ces valeurs expérimentales avec celles précédemment obtenues pour l'acide acrylique, on s'aperçoit que, pour un taux de greffage comparable (de l'ordre de 25 à 30 %), la reprise en humidité des EVA greffés à l'acrylamide est supérieure d'environ 10 à 20 % à celle de leurs homologues acides.

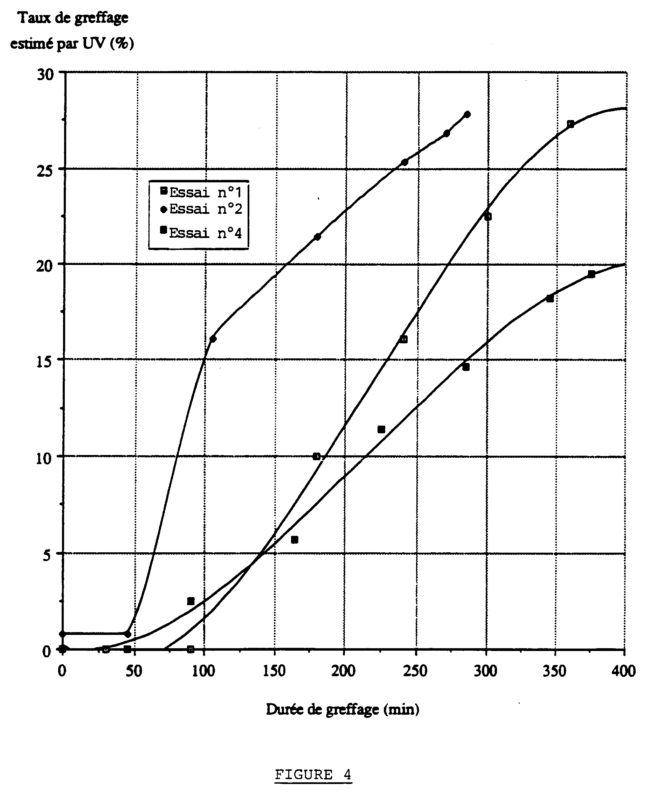

La figure 4 illustre le taux de greffage obtenu (estimé par UV en %) en fonction de la durée de greffage (minutes). On obtient des copolymères greffés plus hydrophiles que ceux obtenus avec l'acide acrylique.

**EXEMPLE 11 : Préparation d'un copolymère EVA/acide acrylique, apte à être utilisé comme film dans un système transdermique.**

L'EVA est directement utilisé sous forme de film (film de 70 µm d'épaisseur).

Le protocole opératoire comprend :

a) l'irradiation de l'échantillon (débit de dose = 5 kGy.h$^{-1}$, dose absorbée : 30 kGy). Source de cobalt[60].

b) le chauffage pendant 8 heures à 60°C en milieu désoxygéné dans une solution aqueuse à 25 % d'acide acrylique et contenant 20 g.l$^{-1}$ de sel de Mohr.

**Revendications**

1. Film, apte à être utilisé comme matrice de principe actif dans un système transdermique, caractérisé en ce qu'il est constitué par un polymère hydrophobe de type éthylène/acétate de vinyle (EVA) contenant au moins un principe actif et comportant 10 à 50 % d'inclusions hydrophiles formées à partir d'au moins un monomère hydrophile choisi dans le groupe qui comprend l'acrylamide, le méthylol acrylamide, le diacétone acrylamide, l'acide maléique, l'acide acrylique, l'acide fumarique, l'acide itaconique, l'acrylate de propylène glycol, le méthacrylate d'éthylène glycol, le méthacrylamide, l'acide méthacrylique, le méthacrylate de propylène glycol, le méthacrylate d'hydroxyéthyle, le méthacrylate de diméthylaminoéthyle, la N-vinylpyrrolidone, l'acide vinylacétique, les acides vinylsulfoniques.

2. Film selon la revendication 1, caractérisé en ce que l'EVA comprend jusqu'à 40 % d'acétate de vinyle, de préférence 14 à 22 %.

**3.** Film selon la revendication 1 ou la revendication 2, caractérisé en ce que lesdites inclusions hydrophiles sont formées à partir d'acide acrylique.

**4.** Film selon la revendication 3, caractérisé en ce que le pourcentage d'acide acrylique est compris entre 22 et 30 %.

**5.** Film selon la revendication 4, caractérisé en ce que ledit copolymère comprend de l'EVA à 14-33 % d'acétate de vinyle, dans lequel 22-30 % d'acide acrylique sont greffés.

**6.** Film selon l'une quelconque des revendications 1 à 5, caractérisé en ce qu'il comprend, en tant que copolymère, de l'EVA à 14-22 % d'acétate de vinyle, dans lequel 26 % d'acide acrylique sont greffés.

**7.** Film selon l'une quelconque des revendications 1 à 5, caractérisé en ce qu'il comprend, en tant que copolymère, de l'EVA à 30-36 % d'acétate de vinyle, dans lequel 26 % d'acide acrylique sont greffés.

**8.** Film selon la revendication 1 ou la revendication 2, caractérisé en ce lesdites inclusions hydrophiles sont formées à partir d'un mélange d'acide acrylique ou d'acrylamide et de N-vinylpyrrolidone.

**9.** Film selon la revendication 1 ou la revendication 2, caractérisé en ce que lesdites inclusions sont formées à partir d'acrylamide.

**10.** Film selon l'une quelconque des revendications 1 à 9, caractérisé en ce que son épaisseur est comprise entre 50 et 500 µm.

**11.** Film selon l'une quelconque des revendications 1 à 10, caractérisé en ce qu'il comprend jusqu'à 40 % de principe actif.

**12.** Film selon la revendication 11, caractérisé en ce qu'il comprend en tant que principe actif, un AINS.

**13.** Film selon la revendication 11, caractérisé en ce qu'il comprend en tant que principe actif, du 17β estradiol, éventuellement associé à un progestatif.

**14.** Film selon la revendication 11, caractérisé en ce qu'il comprend en tant que principe actif, de la physostigmine.

**15.** Film selon la revendication 11, caractérisé en ce qu'il comprend en tant que principe actif, de la trinitrine.

**16.** Film selon la revendication 11, caractérisé en ce qu'il comprend en tant que principe actif, de la nicotine.

**17.** Film selon la revendication 11, caractérisé en ce qu'il comprend en tant que principe actif, de la dihydroergotamine (DHE).

**18.** Film selon la revendication 11, caractérise en ce qu'il comprend en tant que principe actif, de la bromocriptine.

**19.** Film selon la revendication 11, caractérisé en ce qu'il comprend en tant que principe actif, de l'acide salicylique.

**20.** Film selon l'une quelconque des revendications 11 à 19, caractérisé en ce que ledit principe actif est associé à au moins un stimulateur de perméabilité.

**21.** Système transdermique du type comprenant un support occlusif, un réservoir de principe actif, une interface adhésive et un film protecteur, caractérisé en ce que le réservoir de principe actif est constitué par un film selon l'une quelconque des revendications 1 à 19, dans lequel les inclusions hydrophiles sont formées à partir d'au moins un monomère hydrophile choisi dans le groupe qui comprend l'acrylamide, l'acrylate d'éthylène glycol, le méthylol acrylamide, le diacétone acrylamide, l'acide maléique, l'acide acrylique, l'acide fumarique, l'acide itaconique, l'acrylate de propylène glycol, le méthacrylate d'éthylène glycol, le méthacrylamide, l'acide méthacrylique, le méthacrylate de propylène glycol, le méthacrylate d'hydroxyéthyle, le méthacrylate de diméthylaminoéthyle, la N-vinylpyrrolidone, l'acide vinylacétique, les acides vinylsulfoniques.

**22.** Système transdermique selon la revendication 21, caractérisé en ce qu'il comprend :

. un film imperméable comprenant des matériaux utilisés notamment dans l'industrie alimentaire ou pharmaceutique, choisis parmi les dichlorures de polyvinylidène (PVDC), les polyéthylènes (PE), l'éthylène alcool polyvinylique, les polypropylènes, les polyesters, le polychlorotrifluoroéthylène, éventuellement associés sous forme de complexe avec une feuille métallique,

. une matrice de principes actifs, constituée par un film selon l'une quelconque des revendications 1 à 20,

. un adhésif inerte chimiquement vis-à-vis de la matrice de principes actifs et n'interférant pas dans sa capacité à désorber les principes actifs, lequel adhésif est sélectionné parmi les polymères acryliques, le polyuréthane, les silicones, l'EVA et

. une feuille de protection compatible avec l'adhésif sélectionne.

**23.** Système transdermique selon la revendication 22, caractérisé en ce que la matrice de principes actifs, constituée en film, est à base d'un copolymère EVA/AA, de préférence un copolymère EVA 18/AA 26 selon la revendication 6.

**24.** Système transdermique selon la revendication 22, caractérisé en ce que la matrice de principes actifs, constituée en film, est à base d'un copolymère EVA/AAm.

**25.** Système transdermique selon la revendication 22, caractérisé en ce que ledit film comprend en outre un stimulateur de perméabilité dudit principe actif à travers la peau, sélectionné parmi les substances non toxiques, qui ne sont pas un solvant de la matrice polymérique, telles que des alcools et des polyols.

**26.** Système transdermique selon l'une quelconque des revendications 21 à 25, caractérisé en ce qu'il contient au moins deux principes actifs.

**27.** Système transdermique selon la revendication 26, caractérisé en ce lesdits principes actifs sont répartis dans la masse de la matrice polymérique.

**28.** Système transdermique selon la revendication 26, caractérisé en ce que chaque principe actif est dans un segment différent de la matrice polymérique.

**29.** Procédé de préparation d'un film selon l'une quelconque des revendications 1 à 20, apte à servir de matrice à au moins un principe actif, caractérisé en ce qu'il comprend :

(a) la mise en contact de l'EVA, sous forme de poudre, de granulé ou de film, et des monomères hydrophiles,
(b) la polymérisation réticulée desdits monomères hydrophiles et le greffage, sur l'EVA, du polymère hydrophile obtenu (copolymérisation EVA/polymère hydrophile) par irradiation, jusqu'à obtention d'un copolymère EVA/polymère hydrophile, dans lequel le taux de greffage du polymère hydrophile est compris entre 10 et 50 %,
(c) le chargement de la matrice copolymérique en principe actif et s'il y a lieu, c'est-à-dire lorsque l'EVA est sous forme de poudre ou de granulé,
(d) la mise en forme du copolymère obtenu sous la forme d'un film.

**30.** Procédé selon la revendication 29, caractérisé en ce que l'étape (b) de copolymérisation est réalisée en milieu liquide, en présence d'un inhibiteur d'homopolymérisation des monomères hydrophiles.

**31.** Procédé selon la revendication 29 ou la revendication 30, caractérisé en ce que l'étape (b) de copolymérisation est réalisée en milieu liquide, en présence d'agents de réticulation choisis parmi les composés polyinsaturés suivants : le méthylène bis acrylamide, le divinylbenzène, le cyanurate de triallyle, les acrylates ou les méthacrylates d'éthylène, butylène et tetraéthylène glycol, l'orthophosphate de triallyle.

**32.** Procédé selon l'une quelconque des revendications 29 à 31, caractérisé en ce que cette étape (b) est réalisée en irradiant pendant plusieurs heures la solution, par un rayonnement ionisant, la dose totale d'irradiation étant comprise entre 0,5 et 50 KGy, pour l'obtention d'un taux de greffage de polymères hydrophiles compris entre 10 et 50 %.

**33.** Procédé selon l'une quelconque des revendications 29 à 32, caractérisé en ce que l'étape (c) de chargement en principe actif est réalisée avant l'étape (d) de mise en forme.

**34.** Procédé selon l'une quelconque des revendications 29 à 32, caractérisé en ce que l'étape (c) de chargement en principe actif est réalisée après l'étape (d) de mise en forme.

**35.** Procédé selon l'une quelconque des revendications 29 à 34, caractérisé en ce que ladite étape (c) de chargement en principe actif peut être réalisée par l'une quelconque des méthodes suivantes :

. trempage,
. dispersion dans le copolymère,
. diffusion à partir d'un support imprégné en principe actif,
. immersion dans une atmosphère de vapeur saturante.

**36.** Procédé selon l'une quelconque des revendications 29 à 35, caractérisé en ce que l'étape (d) de mise en forme est avantageusement réalisée par extrusion.

**37.** Procédé de préparation du film apte à servir de matrice de principe actif, selon l'une quelconque des revendications 1 à 20, caractérisé en ce qu'il comprend :

(a) l'irradiation de l'EVA, sous forme de poudre, de granulé ou de film, à une dose comprise entre 10 et 80 KGy ;
(b) le stockage de l'EVA irradié pendant plusieurs semaines à température ambiante ou pendant plusieurs mois à basse température ;
(c) la copolymérisation EVA/polymère hydrophile par mise en contact de l'EVA irradié obtenu en (b) avec des monomères hydrophiles pendant plusieurs heures, pour l'obtention d'un copolymère EVA/polymère hydrophile, dans lequel le taux de greffage du polymère hydrophile est compris entre 10 et 50 % ;
(d) le chargement du copolymère en principe actif et s'il y a lieu, c'est-à-dire lorsque l'EVA est sous forme de poudre ou de granulé,
(e) la mise en forme du copolymère obtenu sous la forme d'un film.

**38.** Procédé de préparation d'un copolymère EVA/inclusions hydrophiles, selon la revendication 1, caractérisé en ce qu'il comprend :

(a) l'irradiation de l'EVA, sous forme de poudre, de granulé ou de film, à une dose comprise entre 10 et 80 KGy ;
(b) le stockage de l'EVA irradié pendant plusieurs semaines à température ambiante ou pendant plusieurs mois à basse température ; et
(c) la copolymérisation EVA/polymère hydrophile par mise en contact de l'EVA irradié obtenu en (b) avec des monomères hydrophiles pendant plusieurs heures, pour l'obtention d'un copolymère EVA/polymère hydrophile, dans lequel le taux de greffage du polymère hydrophile est compris entre 10 et 50 %.

**39.** Procédé selon la revendication 38, caractérisé en ce que le copolymère obtenu est chargé en substance chimique, selon l'un des modes de chargement de la revendication 35.

**Claims**

**1.** Film, capable of being used as active principle matrix in a transdermal system, characterized in that it consists of a hydrophobic polymer of ethylene/vinyl acetate (EVA) type containing at least one active principle and composed of 10 to 50% of hydrophilic inclusions formed from at least one hydrophilic monomer chosen from the group which comprises acrylamide, methylolacrylamide, diacetone acrylamide, maleic acid, acrylic acid, fumaric acid, itaconic acid, propylene glycol acrylate, ethylene glycol methacrylate, methacrylamide, methacrylic acid, propylene glycol methacrylate, hydroxyethyl methacrylate, dimethylaminoethyl methacrylate, N-vinylpyrrolidone, vinylacetic acid or vinylsulphonic acids.

**2.** Film according to Claim 1, characterized in that the EVA comprises up to 40% of vinyl acetate, preferably 14 to 22%.

**3.** Film according to Claim 1 or Claim 2, characterized in that the said hydrophilic inclusions are formed from acrylic acid.

**4.** Film according to Claim 3, characterized in that the percentage of acrylic acid is between 22 and 30%.

5. Film according to Claim 4, characterized in that the said copolymer comprises EVA containing 14-33% of vinyl acetate, in which 22-30% of acrylic acid are grafted.

6. Film according to any one of Claims 1 to 5, characterized in that it comprises, as copolymer, EVA containing 14-22% of vinyl acetate, in which 26% of acrylic acid are grafted.

7. Film according to any one of Claims 1 to 5, characterized in that it comprises, as copolymer, EVA containing 30-36% of vinyl acetate, in which 26% of acrylic acid are grafted.

8. Film according to Claim 1 or Claim 2, characterized in that the said hydrophilic inclusions are formed from a mixture of acrylic acid or acrylamide and N-vinylpyrrolidone.

9. Film according to Claim 1 or Claim 2, characterized in that the said inclusions are formed from acrylamide.

10. Film according to any one of Claims 1 to 9, characterized in that its thickness is between 50 and 500 $\mu$m.

11. Film according to any one of Claims 1 to 10, characterized in that it comprises up to 40% of active principle.

12. Film according to Claim 11, characterized in that it comprises, as active principle, an NSAI.

13. Film according to Claim 11, characterized in that it comprises, as active principle, 17$\beta$-ooestradiol, optionally in combination with a progestogen.

14. Film according to Claim 11, characterized in that it comprises, as active principle, physostigmine.

15. Film according to Claim 11, characterized in that it comprises, as active principle, trinitrin.

16. Film according to Claim 11, characterized in that it comprises, as active principle, nicotine.

17. Film according to Claim 11, characterized in that it comprises, as active principle, dihydroergotamine (DHE).

18. Film according to Claim 11, characterized in that it comprises, as active principle, bromocriptine.

19. Film according to Claim 11, characterized in that it comprises, as active principle, salicylic acid.

20. Film according to any one of Claims 11 to 19, characterized in that the said active principle is combined with at least one permeability stimulator.

21. Transdermal system of the type comprising an occlusive support, an active principle reservoir, an adhesive interface and a protective film, characterized in that the active principle reservoir consists of a film according to any one of Claims 1 to 19 in which the hydrophilic inclusions are formed from at least one hydrophilic monomer chosen from the group which comprises acrylamide, ethylene glycol acrylate, methylolacrylamide, diacetone acrylamide, maleic acid, acrylic acid, fumaric acid, itaconic acid, propylene glycol acrylate, ethylene glycol methacrylate, methacrylamide, methacrylic acid, propylene glycol methacrylate, hydroxyethyl methacrylate, dimethylaminoethyl methacrylate, N-vinylpyrrolidone, vinylacetic acid or vinylsulphonic acids.

22. Transdermal system according to Claim 21, characterized in that it comprises:

- an impermeable film comprising materials, used particularly in the food or pharmaceutical industry, chosen from polyvinylidene dichlorides (PVDC), polyethylenes (PE), ethylene/polyvinyl alcohol, polypropylenes, polyesters or polychlorotrifluoroethylene, optionally in combination, in complex form, with a metal sheet,
- an active principle matrix consisting of a film according to any one of Claims 1 to 20,
- an adhesive which is chemically inert with respect to the active principle matrix and which does not interfere in its ability to desorb the active principles, which adhesive is selected from acrylic polymers, polyurethane, silicones or EVA and
- a protective sheet compatible with the adhesive selected.

23. Transdermal system according to Claim 22, characterized in that the active principle matrix, consisting of a film, is based on an EVA/AA copolymer, preferably an EVA 18/AA 26 copolymer according to Claim 6.

24. Transdermal system according to Claim 22, characterized in that the active principle matrix, consisting of a film, is based on an EVA/AAm copolymer.

25. Transdermal system according to Claim 22, characterized in that the said film additionally comprises a stimulator of the permeability of the said active principle through the skin selected from non-toxic substances which are not a solvent of the polymeric matrix, such as alcohols and polyols.

26. Transdermal system according to any one of Claims 21 to 25, characterized in that it contains at least two active principles.

27. Transdermal system according to Claim 26, characterized in that the said active principles are distributed in the body of the polymeric matrix.

28. Transdermal system according to Claim 26, characterized in that each active principle is in a different segment of the polymeric matrix.

29. Process for the preparation of a film according to any one of Claims 1 to 20 capable of being used as a matrix containing at least one active principle, characterized in that it comprises:

(a) bringing EVA, in the powder, granule or film form, and hydrophilic monomers into contact,
(b) the crosslinked polymerization of the said hydrophilic monomers and the grafting, onto the EVA, of the hydrophilic polymer obtained (EVA/hydrophilic polymer copolymerization) by irradiation until an EVA/hydrophilic polymer copolymer is obtained in which the degree of grafting of the hydrophilic polymer is between 10 and 50%,
(c) the charging of the copolymeric matrix with active principle, if necessary, that is to say when the EVA is in the powder or granule form,
(d) the shaping of the copolymer obtained in the form of a film.

30. Process according to Claim 29, characterized in that the copolymerization stage (b) is carried out in liquid medium, in the presence of an inhibiter of homopolymerization of the hydrophilic monomers.

31. Process according to Claim 29 or Claim 30, characterized in that the copolymerization stage (b) is carried out in liquid medium in the presence of crosslinking agents chosen from the following polyunsaturated compounds: methylenebisacrylamide, divinylbenzene, triallyl cyanurate, ethylene, butylene and tetraethylene glycol acrylates or methacrylates, or triallyl orthophosphate.

32. Process according to any one of Claims 29 to 31, characterized in that this stage (b) is perferably carried out by irradiating the solution with an ionizing radiation for several hours, the total irradiation dose being between 0.5 and 50 kGy, in order to obtain a degree of grafting of hydrophilic polymers of between 10 and 50%.

33. Process according to any one of Claims 29 to 32, characterized in that the active principle charging stage (c) is carried out before the shaping stage (d).

34. Process according to any one of Claims 29 to 32, characterized in that the active principle charging stage (c) is carried out after the shaping stage (d).

35. Process according to any one of Claims 29 to 34, characterized in that the said active principle charging stage (c) can be carried out by any one of the following methods:

- steeping,
- dispersion in the copolymer,
- diffusion from a support impregnated with active principle,
- immersion in a saturated vapour atmosphere.

36. Process according to any one of Claims 29 to 35, characterized in that the shaping stage (d) is advantageously carried out by extrusion.

37. Process for the preparation of the film capable of being used as active principle matrix according to any one of Claims 1 to 20, characterized in that it comprises:

(a) the irradiation of the EVA, in the powder, granule or film form, at a dose of between 10 and 80 kGy;

(b) the storage of the irradiated EVA for several weeks at room temperature or for several months at low temperature;

(c) the EVA/hydrophilic polymer copolymerization by bringing the irradiated EVA obtained in (b) into contact with hydrophilic monomers for several hours, in order to obtain an EVA/hydrophilic polymer copolymer in which the degree of grafting of the hydrophilic polymer is between 10 and 50%;

(d) the charging of the copolymer with active principle, if necessary, that is to say when the EVA is in the powder or granule form,

(e) the shaping of the copolymer obtained in the form of a film.

38. Process for the preparation of an EVA/hydrophilic inclusions copolymer according to Claim 1, characterized in that it comprises:

(a) the irradiation of the EVA, in the powder, granule or film form, at a dose of between 10 and 80 kGy;

(b) the storage of the irradiated EVA for several weeks at room temperature or for several months at low temperature; and

(c) the EVA/hydrophilic polymer copolymerization by bringing the irradiated EVA obtained in (b) into contact with hydrophilic monomers for several hours in order to obtain an EVA/hydrophilic polymer copolymer in which the degree of grafting of the hydrophilic polymer is between 10 and 50%.

39. Process according to Claim 38, characterized in that the copolymer obtained is charged with chemical substance according to one of the charging methods of Claim 35.

**Patentansprüche**

1. Film, mit der Eignung, als Matrix für einen Wirkstoff in einem transdermalen System verwendet werden zu können, dadurch **gekennzeichnet**, daß er aus einem hydrophoben Polymeren vom Typ Ethylen/Vinylacetat (EVA) besteht und mindestens einen Wirkstoff enthält und 10 bis 50% hydrophile Einschlüsse, gebildet aus mindestens einem hydrophilen Monomeren, ausgewählt aus der Gruppe umfassend Acrylamid, Methylolacrylamid, Diacetonacrylamid, Maleinsäure, Acrylsäure, Fumarsäure, Itaconsäure, Propylenglykolacrylat, Ethylenglykolmethacrylat, Methacrylamid, Methacrylsäure, Propylenglykolmethacrylat, Hydroxyethylmethacrylat, Dimethylaminoethylmethacrylat, N-Vinylpyrrolidon, Vinylessigsäure, Vinylsulfonsäuren, umfaßt.

2. Film nach Anspruch 1, dadurch **gekennzeichnet**, daß das EVA bis zu 40% Vinylacetat, bevorzugt 14 bis 22%, umfaßt.

3. Film nach Anspruch 1 oder 2, dadurch **gekennzeichnet**, daß die hydrophilen Einschlüsse aus Acrylsäure gebildet sind.

4. Film nach Anspruch 3, dadurch **gekennzeichnet**, daß der Prozentsatz an Acrylsäure zwischen 22 und 30% liegt.

5. Film nach Anspruch 4, dadurch **gekennzeichnet**, daß das Copolymere EVA mit 14 bis 33% Vinylacetat, in dem 22 bis 30% Acrylsäure gepfropft sind, umfaßt.

6. Film nach einem der Ansprüche 1 bis 5, dadurch **gekennzeichnet**, daß er als Copolymeres EVA mit 14 bis 22% Vinylacetat, in dem 26% Acrylsäure gepfropft sind, umfaßt.

7. Film nach einem der Ansprüche 1 bis 5, dadurch **gekennzeichnet**, daß er als Copolymeres EVA mit 30 bis 36% Vinylacetat, in dem 26% Acrylsäure gepfropft sind, umfaßt.

8. Film nach Anspruch 1 oder 2, dadurch **gekennzeichnet**, daß die hydrophilen Einschlüsse aus einem Gemisch von Acrylsäure oder Acrylamid und N-Vinylpyrrolidon gebildet sind.

9. Film nach Anspruch 1 oder 2, dadurch **gekennzeichnet**, daß die Einschlüsse aus Acrylamid gebildet sind.

**10.** Film nach einem der Ansprüche 1 bis 9, dadurch **gekennzeichnet**, daß seine Dicke zwischen 50 und 500 µm liegt.

**11.** Film nach einem der Ansprüche 1 bis 10, dadurch **gekennzeichnet**, daß er bis zu 40% Wirkstoff umfaßt.

**12.** Film nach Anspruch 11, dadurch **gekennzeichnet**, daß er als Wirkstoff ein AINS umfaßt.

**13.** Film nach Anspruch 11, dadurch **gekennzeichnet**, daß er als Wirkstoff 17β Östradiol, gegebenenfalls zusammen mit einem Gestagen, umfaßt.

**14.** Film nach Anspruch 11, dadurch **gekennzeichnet**, daß er als Wirkstoff Physostigmin umfaßt.

**15.** Film nach Anspruch 11, dadurch **gekennzeichnet**, daß er als Wirkstoff Trinitrin umfaßt.

**16.** Film nach Anspruch 11, dadurch **gekennzeichnet**, daß er als Wirkstoff Nikotin umfaßt.

**17.** Film nach Anspruch 11, dadurch **gekennzeichnet**, daß er als Wirkstoff Dihydroergotamin (DHE) umfaßt.

**18.** Film nach Anspruch 11, dadurch **gekennzeichnet**, daß er als Wirkstoff Bromocriptin umfaßt.

**19.** Film nach Anspruch 11, dadurch **gekennzeichnet**, daß er als Wirkstoff Salicylsäure umfaßt.

**20.** Film nach einem der Ansprüche 11 bis 19, dadurch **gekennzeichnet**, daß der Wirkstoff mit mindestens einem permeabilitätsfördernden Mittel assoziiert ist

**21.** Transdermales System vom Typ umfassend einen okklusiven Träger, ein Reservoir für den Wirkstoff, eine adhäsive Grenzschicht und einen Schutzfilm, dadurch **gekennzeichnet**, daß das Reservoir für den Wirkstoff aus einem Film nach einem der Ansprüche 1 bis 19 besteht, in dem die hydrophilen Einschlüsse aus mindestens einem hydrophilen Monomeren, ausgewählt aus der Gruppe umfassend Acrylamid, Ethylenglykolacrylat, Methylolacrylamid, Diacetonacrylamid, Maleinsäure, Acrylsäure, Fumarsäure, Itaconsäure, Propylenglykolacrylat, Ethylenglykolmethacrylat, Methacrylamid, Methacrylsäure, Propylenglykolmethacrylat, Hydroxyethylmethacrylat, Dimethylaminoethylmethacrylat, N-Vinylpyrrolidon, Vinylessigsäure, Vinylsulfonsäuren, gebildet sind.

**22.** Transdermales System nach Anspruch 21, dadurch **gekennzeichnet**, daß es umfaßt:

- einen undurchlässigen Film umfassend normalerweise in der Lebensmittel- oder Arzneimittelindustrie verwendete Substanzen, ausgewählt aus Polyvinylidendichloriden (PVDC), Polyethylenen (PE), Polyvinylethylenalkohol, Polypropylene, Polyestern, Polychlortrifluorethylen, gegebenenfalls assoziiert in Form eines Komplexes mit einer Metallfolie,
- eine Matrix für Wirkstoffe, bestehend aus einem Film nach einem der Ansprüche 1 bis 20,
- einen gegenüber der Matrix der Wirkstoffe chemisch inerten Klebstoff, der dessen Fähigkeit, die Wirkstoffe zu desorbieren, nicht beeinträchtigt, wobei der Klebstoff aus Acrylpolymeren, Polyurethan, Siliconen, EVA gewählt ist und
- eine Schutzfolie, die mit dem ausgewählten Klebstoff kompatibel ist.

**23.** Transdermales System nach Anspruch 22, dadurch **gekennzeichnet**, daß die Matrix der Wirstoffe bestehend aus einem Film, auf einem EVA/AA Copolymeren, bevorzugt einem EVA 18/AA 26 Copolymeren nach Anspruch 6, beruht.

**24.** Transdermales System nach Anspruch 22, dadurch **gekennzeichnet**, daß die Matrix der Wirkstoffe, bestehend aus einem Film, auf einem EVA/AAm Copolymeren beruht.

**25.** Transdermales System nach Anspruch 22, dadurch **gekennzeichnet**, daß der Film weiterhin ein Mittel umfaßt, daß die Permeabilität des Wirkstoffs durch die Haut fördert, ausgewählt aus nichttoxischen Substanzen, die kein Lösungsmittel für die Polymermatrix sind, wie Alkohole und Polyole.

**26.** Transdermales System nach einem der Ansprüche 21 bis 25, dadurch **gekennzeichnet**, daß es mindestens zwei Wirkstoffe enthält.

**27.** Transdermales System nach Anspruch 26, dadurch **gekennzeichnet**, daß die Wirkstoffe in der Masse der Polymermatrix verteilt sind.

**28.** Transdermales System nach Anspruch 26, dadurch **gekennzeichnet**, daß jeder der Wirkstoffe in einem anderen Segment der Polymermatrix vorliegt.

**29.** Verfahren zur Herstellung eines Films nach einem der Ansprüche 1 bis 20, mit der Eignung, als Matrix für mindestens einen Wirkstoff zu dienen, dadurch **gekennzeichnet**, daß man:

(a) EVA in Form eines Pulvers, Granulats oder Films und die hydrophilen Monomeren miteinander in Kontakt bringt,
(b) eine vernetzte Polymerisation der hydrophilen Monomeren und die Pfropfung des so erhaltenen hydrophilen Polymeren auf EVA (Copolymerisation EVA/hydrophiles Polymeres) durch Bestrahlung durchführt, bis ein EVA/hydrophiles Polymeres-Copolymeres erhalten wird, in dem der Pfropfungsgrad des hydrophilen Polymeren zwischen 10 und 50% liegt,
(c) die Copolymermatrix mit Wirkstoff belädt und gegebenenfalls, d.h. wenn das EVA in Form eines Pulvers oder Granulats vorliegt,
(d) das so erhaltene Copolymere in die Form eines Films bringt.

**30.** Verfahren nach Anspruch 29, dadurch **gekennzeichnet**, daß man die Stufe (b) der Copolymerisation in einem flüssigen Medium in Gegenwart eines Homopolymerisationsinhibitors der hydrophilen Monomeren durchführt.

**31.** Verfahren nach Anspruch 29 oder 30, dadurch **gekennzeichnet**, daß die Stufe (b) der Copolymerisation in einem flüssigen Medium in Gegenwart von Vernetzungsmitteln, ausgewählt aus den folgenden mehrfach ungesättigten Verbindungen, durchgeführt wird: Methylenbisacrylamid, Divinylbenzol, Triallylcyanurat, Ethylen-, Butylen- und Tetraethylenglykolacrylate oder -methacrylate, Triallylorthophosphat.

**32.** Verfahren nach einem der Ansprüche 29 bis 31, dadurch **gekennzeichnet**, daß diese Stufe (b) durch Bestrahlen der Lösung während mehrerer Stunden mit einer ionisierenden Strahlung durchgeführt wird, wobei die Gesamtstrahlendosis zwischen 0,5 und 50 KGy liegt, um einen Pfropfungsgrad der hydrophilen Polymeren zwischen 10 und 50% zu erhalten.

**33.** Verfahren nach einem der Ansprüche 29 bis 32, dadurch **gekennzeichnet**, daß die Stufe (c) der Beladung mit Wirkstoff vor der Stufe (d) der Formgebung durchgeführt wird.

**34.** Verfahren nach einem der Ansprüche 29 bis 32, dadurch **gekennzeichnet**, daß die Stufe (c) der Beladung mit dem Wirkstoff nach der Stufe (d) der Formgebung durchgeführt wird.

**35.** Verfahren nach einem der Ansprüche 29 bis 34, dadurch **gekennzeichnet**, daß die Stufe (c) der Beladung mit Wirkstoff nach einem der folgenden Verfahren durchgeführt werden kann:

- Eintauchen,
- Dispersion in dem Copolymeren,
- Diffusion ausgehend von einem mit dem Wirkstoff imprägnierten Träger,
- Eintauchen in eine gesättigte Dampfatmosphäre.

**36.** Verfahren nach einem der Ansprüche 29 bis 35, dadurch **gekennzeichnet**, daß die Stufe (d) der Formgebung vorteilhafterweise durch Extrusion durchgeführt wird.

**37.** Verfahren zur Herstellung des Films, mit der Eignung, als Matrix für einen Wirkstoff zu dienen, nach einem der Ansprüche 1 bis 20, dadurch **gekennzeichnet**, daß man

(a) EVA in Form eines Pulvers, Granulats oder Films mit einer Dosis zwischen 10 und 80 KGy bestrahlt;
(b) das bestrahlte EVA mehrere Wochen bei Umgebungstemperatur oder mehrere Monate bei niedriger Temperatur lagert;
(c) die Copolymerisation EVA/hydrophiles Polymeres durch Inkontaktbringen des in (b) erhaltenen bestrahlten EVA mit hydrophilen Monomeren während mehrerer Stunden durchführt, um ein EVA/hydrophiles Polymeres-Copolymeres zu erhalten, in dem der Pfropfungsgrad des hydrophilen Polymeren zwischen 10 und 50% liegt;

(d) das Copolymere mit dem Wirkstoff belädt und gegebenenfalls, d.h. wenn das EVA in Form eines Pulvers oder Granulats vorliegt,

(e) das so erhaltene Copolymere in Form eines Films bringt.

**38.** Verfahren zur Herstellung eines Copolymeren aus EVA/hydrophilen Einschlüssen nach Anspruch 1, dadurch **gekennzeichnet**, daß man

(a) das EVA in Form eines Pulvers, Granulats oder Films mit einer Dosis zwischen 10 und 80 KGy bestrahlt;

(b) das bestrahlte EVA mehrere Wochen bei Umgebungstemperatur oder mehrere Monate bei niedriger Temperatur lagert und

(c) die Copolymerisation EVA/hydrophiles Copolymeres durch Inkontaktbringen des in (b) erhaltenen bestrahlten EVA mit hydrophilen Monomeren während mehrerer Stunden durchführt, um ein EVA/hydrophiles Polymeres-Copolymeres zu erhalten, in dem der Pfropfungsgrad des hydrophilen Polymeren zwischen 10 und 50% beträgt.

**39.** Verfahren nach Anspruch 38, dadurch **gekennzeichnet**, daß das erhaltene Copolymere mit einer chemischen Substanz nach einer der Beladungsmethoden nach Anspruch 35 beladen wird.

```
┌─────────────────┐                        ┌─────────────────┐
│                 │                        │                 │
│    EVA  18      │                        │  Acide Acrylique│
│                 │                        │                 │
└────────┬────────┘                        └────────┬────────┘
         │                                          │
         ▼                                          │
┌─────────────────┐                                 │
│   Irradiation   │              H₂O       Additifs │
│  20 < dose < 30 │               │           │     │
│     (KGy)       │               ▼           ▼     ▼
└────────┬────────┘              ┌─────────────────────────┐
         │                       │                         │
         ▼                       │     Solution mère       │
┌─────────────────┐              │                         │
│    Stockage     │              └────────────┬────────────┘
│(< 14 jours à 20°C)│                          │
└────────┬────────┘                            │
         │                                     │
         └──────────────┐       ┌──────────────┘
                        ▼       ▼
                  ┌─────────────────┐
                  │                 │
                  │    Greffage     │
                  │                 │
                  └────────┬────────┘
                           ▼
                  ┌─────────────────┐
                  │                 │
                  │     Lavage      │
                  │                 │
                  └────────┬────────┘
                           ▼
                  ┌─────────────────┐      ┌─────────────────────┐
                  │                 │      │    Caractérisation  │
                  │     Séchage     │─────▶│   physico-chimique  │
                  │                 │      │                     │
                  └────────┬────────┘      └─────────────────────┘
                           ▼
                  ┌─────────────────┐
                  │                 │
                  │   Utilisation   │
                  │                 │
                  └─────────────────┘
```

FIGURE 1

FIGURE 2

FIGURE 3

EP 0 625 901 B1

FIGURE 4